# EUROPÄISCHE PATENTSCHRIFT

(11) **EP 1 868 574 B1**
(45) Veröffentlichungstag und Bekanntmachung des Hinweises auf die Patenterteilung: **06.01.2010**
(21) Anmeldenummer: 06724283.4
(22) Anmeldetag: 12.04.2006
(51) Int. Cl.: A61K 9/14, A61K 9/51

(54) **VERFAHREN ZUR SCHONENDEN HERSTELLUNG HOCHFEINER PARTIKELSUSPENSIONEN UND HOCHFEINER PARTIKEL SOWIE DEREN VERWENDUNG**
METHOD FOR PRODUCING ULTRAFINE PARTICLE SUSPENSIONS UNDER MILD CONDITIONS, ULTRAFINE PARTICLES AND USE THEREOF
PROCÉDÉ DE PRODUCTION DE SUSPENSIONS DE PARTICULES TRÈS FINES EN CONDITIONS MODÉRÉES, PARTICULES TRÈS FINES CORRESPONDANTES ET LEUR UTILISATION

(30) Priorität: 13.04.2005 DE 102005017777; 05.11.2005 DE 102005053462
(43) Veröffentlichungstag der Anmeldung: 26.12.2007
(73) Patentinhaber: Abbott GmbH & Co. KG, 65205 Wiesbaden (DE)
(72) Erfinder: MÖSCHWITZER, Jan, 12247 Berlin (DE); LEMKE, Andreas, 12203 Berlin (DE)
(74) Vertreter: UEXKÜLL & STOLBERG
(86) Internationale Anmeldenummer: PCT/EP2006/003377
(87) Internationale Veröffentlichungsnummer: WO 2006/108637

(56) Entgegenhaltungen:
- WO-A2-2006/094808
- DE-A1- 19 932 157
- TRACY M A: "Development and scale-up of a microsphere protein delivery system" BIOTECHNOLOGY PROGRESS, Bd. 14, Nr. 1, Januar 1998 (1998-01), Seiten 108-115, XP002312915 ISSN: 8756-7938
- WOLF M ET AL: "Stabilisation and determination of the biological activity of L-asparaginase in poly(D,L-lactide-co-glycolide) nanospheres." INTERNATIONAL JOURNAL OF PHARMACEUTICS 30 APR 2003, Bd. 256, Nr. 1-2, 30. April 2003 (2003-04-30), Seiten 141-152, XP002421906 ISSN: 0378-5173

## Beschreibung

### 1. Gebiet der Erfindung

Die Erfindung beschreibt ein Verfahren zur schonenden Herstellung hochfeiner Partikelsuspensionen und hochfeiner Partikel, deren Partikel/die eine durchschnittliche Größe im Nanometerbereich aufweisen, für Bereiche der Pharmazie, Kosmetik, Lebensmittelherstellung und Agrar.

### 2. Stand der Technik

Aufgrund der heute angewendeten Techniken zur Findung neuer Arzneistoffkandidaten (z.B. high-throughput-screening, molecular modelling, receptor-fit-techniques) (B. Rabinow, Nanosuspensions in drug delivery, Nat. Rev. Drug Discov. 9/2004, 3(9), 785-796), sind immer mehr Wirkstoffe, die aus der pharmazeutischen Entwicklung kommen, zwar besonders gut wirksam, besitzen dabei aber häufig nur eine sehr geringe Löslichkeit bzw. sind praktisch unlöslich (Merisko-Liversidge E. Nanocrystals: Resolving Pharmaceutical Formulation Issues associated with poorly water-soluble Compounds. In: Marty JJ, editor. Particles; 2002; Orlando: Marcel Dekker; 2002). Dadurch wird ihre Bioverfügbarkeit, besonders nach oraler oder topischer Applikation, deutlich limitiert. Eine parenterale Anwendung wird durch die schlechte Löslichkeit und die damit verbundenen erforderlichen großen Injektionsvolumina ebenfalls erschwert. Die Verwendung von injizierbaren Lösungsmittelmischungen (z.B. Wasser-Ethanol-Gemischen) oder organischen Lösungsmitteln (z.B. Polyethylenglycol), auch unter Zuhilfenahme von Lösungsvermittlern führt häufig zu schmerzhaften Injektionen und ist deshalb ebenfalls negativ zu bewerten.

Einen möglichen Ansatz zur Verbesserung der Bioverfügbarkeit aufgrund einer gesteigerten Auflösungsgeschwindigkeit und einer erhöhten Sättigungslöslichkeit bietet die Nanonisierung, das heißt die Verringerung der Partikelgröße in einen Bereich kleiner als 1000 nm. (Merisko-Liversidge E, Liversidge GG, Cooper ER. Nanosizing: a formulation approach for poorlywater-soluble compounds. European Journal of Pharmaceutical Sciences 2003;18(2):113-120.) Die kleine Partikelgröße führt einerseits zu einer stark vergrößerten Gesamtoberfläche und andererseits zu einer stärkeren Krümmung der Partikeloberfläche. Dadurch kommt es zu einem gesteigerten Lösungsdruck entsprechend der Kelvin-Gleichung und einer damit verbundenen Steigerung der Sättigungslöslichkeit. Die Steigerung der Sättigungslöslichkeit und die stark vergrößerte Oberfläche führen entsprechend der Noyes-Whitney-Gleichung zu einer erhöhten Auflösungsgeschwindigkeit. Dementsprechend stehen durch Nanonisierung von Arzneistoffen verglichen mit mikronisiertem Arzneistoff in kürzerer Zeit größere Mengen an gelöstem Wirkstoff zur Verfügung, wodurch im Falle von BSC (engl.: biopharmaceutical specification class (BSC)) Klasse II Arzneistoffen, die Bioverfügbarkeit deutlich verbessert werden kann.

Klasse II (BSC II) Arzneistoffe sind solche, die nach peroaler Gabe zwar leicht permieren, deren Bioverfügbarkeit aber aufgrund einer langsamen Auflösungsgeschwindigkeit/ge-ringen Sättigungslöslichkeit deutlich limitiert ist.

Es sind verschiedenste Methoden beschrieben, um Wirkstoffe mit einer Partikelgröße im Nanometerbereich herzustellen. Prinzipiell unterscheidet man zwischen "bottom-up" und "top-down" Technologien. Bei den "top-down"-Technologien geht man von größeren Arzneistoffkristallen aus, die meist in einem ersten Produktionsschritt mit Hilfe von Mahlverfahren (wie z.B. dem Luftstrahlmahlen) mikronisiert werden. Bei der Verwendung von "top-down"-Technologien geht man im Allgemeinen davon aus, dass eine vorherige Mikronisierung des Ausgangsmaterials zu einer besseren Nanonisierung führt. (V.B. Patravale, Nanosuspensions: a promising drug delivery strategy, Journal of Pharmacy and Pharmacology, 56(7) 827-840).

Für die eigentliche Nanonisierung werden verschiedene Techniken beschrieben.

Die US-A-5 145 684 beschreibt die Naßmahlung von Arzneistoffen mit Kugelmühlen, um die Größe von Arzneistoffkristallen dispergiert in Tensidlösungen zu reduzieren. Die Partikelgröße der "Makrosuspension" wird durch die Mahlkugeln und deren Bewegung reduziert. Ein Nachteil dieser Technologie ist die Notwendigkeit der Verwendung von mikroniserten Ausgangsmaterialien, eine mögliche Kontamination des Produktes durch Abrieb von den Mahlkugeln (Buchmann S, Fischli, W., Thiel, F. P., Alex, R. Aqueous microsuspension, an alternative intravenous formulation for animal studies. In: 42 nd Annual Congress of the International Association for Pharmaceutical Technology (APV); 1996; Mainz; 1996. p. 124) und die deutliche Abhängigkeit des Mahlergebnisses und der erforderlichen Mahldauer von den Stoffeigenschaften des Ausgangsmaterials. Die erzielbaren Partikelgrößen liegen in Abhängigkeit von dem Mahlgut typischerweise unter 400 nm; häufig kann eine Partikelgröße von 200-300 nm erreicht werden. Um Partikelgrößen im Bereich von 100 nm oder darunter zu erzielen, sind jedoch sehr lange Mahlzeiten und spezielle Techniken (z.B. Wechseln der Kugelgröße) erforderlich, was die Prozessführung erschwert und deutlich verlängert.

Eine alternative Herstellungsmethode ist die Verwendung von Hochdruckhomogenisatoren, also Methoden, die auf dem Kolben-Spalt-Prinzip oder dem Jet-Stream-Prinzip (Microfluidizer-Technologie, Microfluidics Inc. (US-A-6 018 080)) beruhen. Prinzip des Microfluidizers ist das frontale Aufeinanderprallen zweier Strahlen mit sehr großer Geschwindigkeit, wobei die Kollision der Partikel zu deren Zerkleinerung führt. Nachteile dieser Methode sind die erforderliche Zyklenzahl (häufig mehr als 50 Zyklen) und eine potentielle Kontamination mit zurückbleibenden Mikropartikeln.

Bei der Verwendung von Kolben-Spalt-Homogenisatoren wird die Makrosuspension durch einen sehr engen Spalt gepresst, der in Abhängigkeit vom aufgewendeten Druck und von der Viskosität des Dispersionsmediums eine Größe von 5-20 µm aufweist (Rainer H. Müller, Jan Möschwitzer and Faris Nadiem Bushrab, Manufacturing of nanoparticles by milling and homogenization techniques, eds. Gupta, Kompella, Publisher: Marcel Dekker, submitted for printing). Dabei führt die hohe Strömungsgeschwindigkeit zu Kavitationskräften, zusätzlich führen Partikelkollision sowie auftretende Scherkräfte ebenfalls zu einer Partikelzerkleinerung. Das Patent US-A-5 858 410 beschreibt die Verwendung von Kolben-Spalt-Homogenisatoren zur Zerkleinerung von in reinen Wasser-Tensid-Mischungen dispergierten Partikeln. Die WO-A-0103670 beschreibt dagegen die Verwendung dieser Technik, um Partikel, die in nichtwässrigen Medien oder in Mischungen von Wasser mit mit Wasser mischbaren Flüssigkeiten dispergiert sind, zu homogenisieren. Die mit Kolben-Spalt-Homogenisatoren erzielbaren Partikelgrößen liegen dabei an Abhängigkeit von der Größe und Eigenschaften der verwendeten Ausgangsmaterialien sowie den verwendeten Dispersionsmedien und der eingebrachten Leistungsdichte im Bereich von ca. 200-600 nm und im Fall von sehr harten Materialien im Bereich von ungefähr 700-900 nm (Muller RH, Jacobs C, Kayser O. Nanosuspensions as particulate drug formulations in therapy: Rationale for development and what we can expect for the future. Advanced Drug Delivery Reviews 2001;47(1):3-19;).

Mit den oben beschriebenen "top-down"-Techniken ist es bis heute kaum bzw. unmöglich, bei vertretbarem Aufwand Nanosuspensionen mit einer mittleren Partikelgröße von weit unter 100 nm und einer maximalen Partikelgröße im Bereich von 100-200 nm herzustellen.

Bei der Verwendung der sogenannten "bottom-up"-Technologien geht man von Arzneistofflösungen aus, also molekular feinstverteilten Arzneistoffmolekülen. Gibt man diese Lösung entsprechend schnell zu einem Nichtlösungsmittel, das aber mit dem verwendeten Lösungsmittel des ersten Schrittes mischbar ist, fallen sehr kleine Wirkstoffkristalle aus, die aber mit der Zeit zu stabileren, größeren Kristallen anwachsen. Diese Methode ist schon sehr alt und wird als "via humida paratum" (auf flüssigem Wege bereitet) bezeichnet.

Um das Wachstum der Partikel zu verlangsamen werden im Allgemeinen Tenside oder polymere Stabilisatoren verwendet. Diese Technik wird als Hydrosol-Technik bezeichnet und in der US-A-5 389 382 beschrieben. Später wurden einige Modifikationen dieses Präzipitationsprinzipes beschrieben (siehe US-A-6 251 945). Das Hauptproblem ist, die präzipitierten Kristalle in Nanometerbereich zu stabilisieren. Die Nanokristalle versuchen zu wachsen und Mikrokristalle zu bilden. Um dies zu verhindern, kann man die sofortige Trocknung der hergestellten Suspension, z.B. durch Lyophilisation (Sucker, H., Hydrosole - eine Alternative für die parenterale Anwendung von schwer wasserlöslichen Wirkstoffen, in: Müller, R. H., Hildebrand, G. E., (Hrsg.), Pharmazeutische Technologie: Moderne Arzneiformen, 2. Auflage, 1998, WVG, Stuttgart) anwenden. Ein alternativer Ansatz ist die Fällung der Partikel mit anschließendem Eintrag von Energie (z.B. durch Scherkräfte oder Ultraschall (US-A-6 607 784). Diese Kräfte können z.B. durch Hochgeschwindigkeitsmischer oder verschiedene Hochdruckhomogenisatoren (z.B. Geräte der Firmen APV Gaulin, NiroSoavi, Avestin) oder im Fall der Ultraschallverwendung durch Geräte der Fima Sonics aufgebracht werden. Durch die Behandlung der ausgefällten Partikel mit solchen Kräften wird eine Stabilisierung der Partikelgröße erreicht, die Kristalle verändern ihre Größe während der Lagerung nicht oder nur unwesentlich, im Gegensatz zu den Kristallen, die nicht mit Scherkräften behandelt wurden. Ein Nachteil dieser Technik (US-A-6 607 784) ist, dass es - zumindest in den meisten Fällen - erforderlich ist, dass Lösungsmittel zu entfernen. Außerdem können nur Wirkstoffe prozessiert werden, für die es mindestens ein gutes Lösungsmittel und ein Nichtlösungsmittel gibt, das mit dem Lösungsmittel mischbar ist. Ein weiterer Nachteil ist, dass im Allgemeinen jedes Lösungsmittel zumindest in einem bestimmten Umfang in dem Nicht-Lösungsmittel (z.B. Wasser) löslich ist, das bedeutet, dass bei nachträglicher Entfernung des verwendeten Lösungsmittels immer ein gewisser Restgehalt desselben im Wasser zurückbleibt. Im Gegensatz zu der Lehre der US-A-6 607 784, bei der die Ausfällung des schwerlöslichen Wirkstoffs vor dem Aufbringen von Kraft erfolgt, wird in der Patentanmeldung US-A-2004/0266890 eine Technik beschrieben, bei der der Mischvorgang der Flüssigkeiten und das Aufbringen der Kraft in einem speziell dafür konstruiertem Gerät erfolgt. Dafür ist es erforderlich, das die verwendeten Flüssigkeitsströme in einer besonderen Anordnung zueinander stehen. Die bei der Verwendung dieser neuen Technologie, speziell in der gleichzeitigen Variante (4. Prozesskategorie), erzielbaren Partikelgrößen wurden nicht bestimmt. Es werden aber Partikelgrößen im Bereich von 10 nm bis 10 µm angegeben, ohne spezielle Bespiele für die beanspruchten 10 nm aufzuführen.

Aus den aufgeführten Beispielen wird deutlich, dass mit den bis dato bekannten Methoden eine rationelle Herstellung von lager- und langzeitstabilen, hochfeinen Nanosuspensionen mit einer mittleren Partikelgröße im Bereich von 50 nm bis unter 1000 nm, bevorzugt 50 nm bis 600 nm, besonders bevorzugt von 50 nm bis 200 nm derzeit nur relative schwer und unter hohem Kraft- bzw. Energieaufwand erreicht werden kann.

Die vorliegende Erfindung betrifft demgegenüber eine Methode, mit deren Hilfe die oben aufgeführten Probleme gelöst werden können.

Die vorliegende Erfindung beschreibt einen mehrstufigen Prozess, bei dem ein in Wasser schwer bzw. unlöslicher Feststoff in einem geeigneten Lösungsmittel aufgelöst wird, die entstandene Lösung anschließend eingefroren wird, die entstandene gefrorene feste Matrix in einer ersten Ausführungsvariante anschließend beispielsweise durch Gefriertrocknung (Lyophilisation) vom verwendeten Lösungsmittel ganz oder teilweise befreit wird bzw. in einer zweiten Ausführungsvariante die gefrorene feste Matrix ohne Trocknung weiterverarbeitet wird. Die erhaltene feste Matrix, gefroren oder lyophilisiert, wird in einem Dispersionsmittel (äußere Phase) dispergiert. Auf diese Dispersion werden Kräfte (z.B. Ultraschall, Kavitations- und/oder Scherkräfte) angewendet, so dass eine Suspension mit einer mittleren Partikelgröße im Bereich von 50 nm bis unter 1000 nm entsteht, die entweder selbst als Produkt dient oder weiterverarbeitet wird.

Das erfindungsgemäße Verfahren zur schonenden Herstellung von hochfeinen Partikelsuspensionen gemäß Anspruch 1 ist **dadurch gekennzeichnet, dass**
a) ein in Wasser unlöslicher oder in Wasser schwerlöslicher Feststoff in einem geeigneten Lösungsmittel gelöst wird,
b) die Lösung aus a) anschließend unter Bildung einer festen Matrix eingefroren wird,
c) gegebenenfalls der in b) gebildeten festen Matrix in gefrorenem Zustand das Lösungsmittel durch Trocknung, insbesondere Lyophilisation, entzogen wird,
d) die in b) gebildete feste Matrix, die ggf. gemäß c) getrocknete, insbesondere lyophilisiert, worden ist, in einem Dispergiermittel in gefrorenem Zustand dispergiert wird, und
e) anschließend auf die in d) hergestellte Dispersion vor dem Schmelzen der gefrorenen, dispergierten, festen Martix mittlere bis hohe Kräfte aufgebracht werden, so dass eine Partikelsuspension entsteht, deren mittlere Partikelgröße, bestimmt mittels Photonenkorrelationsspektroskopie (PCS), unter 1000 nm, insbesondere im Bereich von 50 bis < 1000 nm, bevorzugt unter 800 nm, vorzugsweise im Bereich von 50 bis 600 nm, und insbesondere unter 400, bevorzugt im Bereich von 50 bis 200 nm, und speziell unter 100 nm liegt.

Bevorzugte Ausführungsformen sind Gegenstand der Unteransprüche.

Die Erfindung umfasst insbesondere gemäß einer weiteren bevorzugten Ausführungsform ein Verfahren zur besonders effektiven, tensidfreien Herstellung von oberflächenmodifizierten Wirkstoffnanopartikeln mit Hilfe der Hochdruckhomogenisation.

Die Herstellung von Wirkstoffnanopartikeln besitzt zunehmende wirtschaftliche Bedeutung, insbesondere wenn es sich bei den Wirkstoffnanopartikeln (allgemeine Bezeichung für Wirkstoffpartikel mit einer mittleren Partikelgrösse von <1000 nm) um Arzneistoffnanokristalle handelt.

Als Nanokristalle (allgemein Wirkstoffnanokristall, speziell Arzneistoffnanokristall) bezeichnet man kristalline, feste Partikel mit einer mittleren Partikelgrösse von 1 bis 1000 nm. Je nach Herstellungsmethode kann es sich auch um Nanopartikel mit teilweise amorphen Bereichen handeln. Im Folgenden werden die Begriffen Wirkstoffnanopartikel und Arzneistoffnanokristall synonym benutzt.

Dispersionen, die Wirkstoffnanopartikel in einer flüssigen Phase dispergiert enthalten, werden im Folgenden auch als Nanosuspensionen bezeichnet.

Die Oberfläche dieser Wirkstoffnanokristalle/Arzneistoffnanokristalle kann gemäß dieser bevorzugten Ausführungsform (mit Oberflächenmodifizierung) mit gegensätzlich geladenen Polyelektrolytschichten überzogen werden, dann dienen die Wirkstoffnanopartikel bzw. Arzneistoffnanokristalle als Templatpartikel.

Die Erfindung umfasst auch die Verwendung der hergestellten Suspensionen bzw. der darin enthaltenen Partikel zur pharmazeutischen und kosmetischen Applikation, vorzugsweise in Form von Tabletten und Kapseln, Cremes, Salben oder Pulvern zur Rekonstitution vor der Anwendung bzw. zur Herstellung von pharmazeutischen und kosmetischen Präparaten, vorzugsweise in Form von Tabletten und Kapseln, Cremes, Salben oder Pulvern zur Rekonstitution vor der Anwendung.

Der zu verarbeitende bzw. zu lösende Feststoff ist insbesondere ein Arzneimittelwirkstoff, ein kosmetischer Wirkstoff, ein Zusatzstoff für Nahrungsmittel, ein Farbstoff oder ein Pigment.

Die in Schritt e) angewendeten mittleren bis hohen Kräfte sind insbesondere Scher-, Kavitations-, Mahl- und/oder Ultraschallkräfte, die insbesondere duch Hochdruckhomogenisatoren, Jet-Stream-Geräte, Rotor-Stator-Kolloidmühlen, Kugelmühlen, Hochscherungsmischer oder Ultraschallapparaturen aufgebracht werden, wobei das jeweils eingesetzte Gerät vorzugsweise mit einer Leistungsdichte von 10⁶ bis 10¹³/m³ arbeitet, insbesondere im Bereich von 10⁹ bis 10¹³/m³.

Für die Auflösung des in Wasser unlöslichen oder schwerlöslichen Feststoffs eingesetzten Lösungsmittel umfassen hydrophile Flüssigkeiten, insbesondere Alkohole, bevorzugt Methanol, Ethanol und Isopropanol, Mischungen von Wasser mit mit Wasser vollständig oder teilweise mischbaren Flüssigkeiten oder hydrophilen Flüssigkeiten, insbesondere Alkoholen, bevorzugt Methanol, Ethanol oder Isopropanol oder anderen organischen Lösungsmitteln, oder mit Wasser nicht mischbare Flüssigkeiten, insbesondere Chloroform oder Dichlormethan, wobei bevorzugte Lösungsmittel, N-Methyl-2-pyrrolidinon, 2-Pyrrolidon, Dimethylacetamid, Ethanol, Methanol, Isopropanol, Aceton, Chloroform, Dichlormethan, Dimethylsulfoxid, N-Propanol, Glycerin, Ethylenglycol, Dimethylformamid, Dimethylacetamid oder Säuren und Basen, insbesondere Chlorwasserstoffsäure, Schwefelsäure, Essigsäure, Ameisensäure, Fumarsäure, Triethanolamin, Pyridin, Ammoniak sind, wobei gegebenenfalls eine Mischung aus zwei oder mehr derselben eingesetzt wird.

Die in a) hergestellte Feststofflösung kann einen oder mehrere weitere Hilfsstoffe und/oder dispersionsstabilisierende Substanzen enthalten, insbesondere Tenside, Stabilisatoren vom Typ der Antiflokkulantien und Polymere, sowie inerte Füllstoffe, wobei die Konzentrationen pro Komponente, bezogen auf das Gewicht, bevorzugt im Bereich von 1-90%, insbesondere von 1-20% und bevorzugt unterhalb von 10% liegen, idealerweise unterhalb von 0,01-5% liegen.

Typische Tenside oder stabilisierende Substanzen, die dem Lösungsmittel zugesetzt werden können, sind z.B. Verbindungen aus der Reihe der Poloxamere, Poloxamine, ethoxylierten Mono-und Diglyceride, ethoxylierten Lipide und Lipoide, ethoxylierten Fettalkohole und Alkylphenole, ethoxylierten Fettsäureester, Polyglycerinether und -ester, Lecithine, Ester und Ether von Zuckern oder Zuckeralkoholen mit Fettsäuren oder Fettalkoholen, Phospholipide und Sphingolipide, Sterine, deren Ester oder Ether sowie deren Mischungen dieser Verbindungen. Daneben kommen auch Eilecithin, Sojalecithin oder hydrierte Lecithine, deren Mischungen oder Mischungen aus einem oder beiden Lecithinen mit einer oder mehreren Phopholipidkomponenten, Cholesterin, Cholesterinpalmitat, Stigmasterin oder andere Sterine in Frage, um der Lösung zugesetzt zu werden.

Unter Umständen kann es erforderlich sein, der Lösung weitere Substanzen zuzusetzen, um die Eigenschaften der Lösung selbst oder die Eigenschaften der aus der Lösung hergestellten festen Matrix zu beeinflussen. Dazu kommen unter anderem in Frage: Diacetylphosphat, Phosphatidylglycerol, gesättigte oder ungesättigte Fettsäuren, Natriumcholat, Antiflokkulantien oder Aminosäuren, sowie Celluloseether und -ester, Polyvinylderivate, Alginate, Xanthane, Pektine, Polyacrylate, Poloxamere und Poloxamine, Polyvinlyalkohol, Polyvinylpyrrolidon oder Glucose, Mannose, Trehalose, Mannit und Sorbit, Fructose, Natriumcitrat, Natriumhydrogenphosphat, Natriumdihydrogenphosphat, Natriumchlorid, Kaliumchlorid und Glycerin. Wenn es erforderlich ist, können dem Lösungsmittel auch Farbstoffe, entweder in gelöster Form oder in unlöslicher Form als Pigmente, zugesetzt werden.

Dieser Lösung, die einen oder mehrere gelöste Stoffe enthält und zusätzlich einen oder mehrere Hilfsstoffe enthalten kann, wird dann in einem schnellen Schritt Wärme entzogen, so dass eine vollständig durchgefrorene Matrix entsteht. Dies kann z.B. durch Einbringen dieser Lösung in flüssigen Stickstoff geschehen, was aufgrund der niedrigen Temperatur von ca. minus 195°C zu einem sofortigen Gefrieren der Lösung führt.

Die zu verarbeitenden Feststoffe können aus verschiedensten Bereichen stammen, d.h. es können pharmazeutische Wirkstoffe, kosmetische Wirkstoffe, aber auch Zusatzstoffe für die Nahrungsmittelindustrie sowie Materialen für andere technische Bereiche verarbeitet werden, die bevorzugt als feinkristallines Material (z.B. mikronisiert, z.B. Partikelgröße im Bereich von 1 - 10 µm) vorliegen sollen, wie z.B. Farbstoffe und Farbstoffpigmente für Farben und Lacke oder für kosmetische Anwendungen.

Pharmazeutische Wirkstoffe können aus den im Folgenden aufgeführten therapeutischen Gebieten stammen (ggf. in Form ihrer wenig wasserlöslichen Form, z. B. als Base anstelle des Hydrochlorids):

Beispiele für zu einer Nanosuspension zu verarbeitende Arzneistoffgruppen sind:
1. Analgetika/Antirheumatika
   z.B. Morphin, Codein, Piritramid, Fentanyl, Levomethadon, Tramadol, Diclofenac, Ibuprofen, Dexibuprofen, Ketoprofen, Dexketoprofen, Meloxicam, Indometacin, Naproxen, Piroxicam, Rofecoxib, Celecoxib,
2. Antiallergika
   z.B. Pheniramin, Dimetinden, Terfenadin, Astemizol, Loratidin, Desloratadin, Doxylamin, Meclozin, Fexofenadin, Mizolastin,
3. Antibiotika/Chemotherapeutika
   z.B. Rifamoicin, Ethambutol, Thiazetazon, Buparvaquon, Atovaqon, Tarazepid,
4. Antiepileptika
   z.B. Carbamazepin, Clonazepam, Mesuximid, Phenytoin, Valproinsäure,
5. Antimykotika
   a) intern:
      z.B. Natamycin, Amphotericin B, Miconazol,
      Itraconazol
   b) extern ausserdem:
      z. B. Clotrimazol, Econazol, Fenticonazol, Bifonazol, Ketoconazol, Tolnaftat,
6. Corticoide (Interna)
   z.B. Aldosteron, Fludrocortison, Betametason, Dexametason, Triamcinolon, Triamcinolonacetonid, Fluocortolon, Hydrocortison, Hydrocortisonacetat, Prednisolon, Prednyliden, Cloprednol, Budesonid, Methylprednisolon,
7. Dermatika
   a) Antibiotika:
      z.B. Tetracyclin, Erythromycin, Framycetin, Tyrothricin, Fusidinsäure
   b) Virustatika wie oben, ausserdem:
      z.B. Vidarabin,
   c) Corticoide wie oben, ausserdem:
      z.B. Amcinonid, Flupredniden, Alclometason, Clobetasol, Halcinonid, Fluocinolon, Clocortolon, Flumetason, Diflucortolon, Fludroxycortid, Halometason, Desoximetason, Fluocinolid, Fluocortinbutyl, Flupredniden, Prednicarbat, Desonid,
8. Hypnotika, Sedativa
   z.B. Cyclobarbital, Pentobarbital, Methaqualon, Benzodiazepine (Flurazepam, Midazolam, Nitrazepam, Lormetazepam, Flunitrazepam, Triazolam, Brotizolam, Temazepam, Loprazolam),
9. Immuntherapeutika und Zytokine
   z.B. Azathioprin, Ciclosporin,
10. Lokalanaesthetika
   a)intern:
      z.B. Butanilicain, Mepivacain, Bupivacain, Etidocain, Lidocain, Articain
   b)extern ausserdem:
      z.B. Oxybuprocain, Tetracain, Benzocain,
11. Migränemittel
   z.B. Lisurid, Methysergid, Dihydroergotamin, Ergotamin, Triptane (wie z.B. Zolmitriptan, Sumatriptan, Rizatriptan),
12. Narkosemittel
   z.B. Methohexital, Propofol, Etomidat, Ketamin, Thiopental, Droperidol, Fentanyl,
13. Nebenschilddrüsenhormone, Calciumstoffwechselregulatoren
   z.B. Dihydrotachysterol,
14. Ophthalmika
   z.B. Cyclodrin, Cyclopentolat, Homatropin, Tropicamid, Pholedrin, Edoxudin, Aciclovir, Acetazolamid, Diclofenamid, Carteolol, Timolol, Metipranolol, Betaxolol, Pindolol, Bupranolol, Levobununol, Carbachol,
15. Psychopharmaka
   z.B. Benzodiazepine (Lorazepam, Diazepam), Clomethiazol,
16. Sexualhormone und ihre Hemmstoffe
   z.B. Anabolika, Androgene, Antiandrogene, Gestagene, Estrogene, Antiestrogene,
17. Zytostatika und Metastasehemmer
   a) Alkylantien wie Melphalan, Carmustin, Lomustin, Cyclophosphamid, Ifosfamid, Trofosfamid, Chlorambucil, Busulfan, Prednimustin, Thiotepa
   b) Antimetabolite wie Fluorouracil, Methotrexat, Mercaptopurin, Tioguanin
   c) Alkaloide wie Vinblastin, Vincristin, Vindesin,
   d) Antibiotoka wie Dactinomycin,
   e) Taxol und verwandte bzw. analoge Verbindungen,
   f) Dacarbazin, Estramustin, Etoposid
   g) Oxalipantin,
   h) Platinverbindungen, z.B. Cisplatin und Carboplatin,
18. Sartane
   Olmesartan, Candesartan, Valsartan, Losartan
19. Fibrate
   Bezafibrat, Fenofibrat; Etofibrat, Etofyllinclofibrat,
20. Statine
   Pravastatin, Simvastatin, Cerivastatin, Atorvastatin, Fluvastatin, Lovastatin, Rosuvastatin,
21. HIV-Therapeutika
   Abacavir, AZT, Aciclovir, Aldesleukin, Amprenavir, Atazanavir, Atovaquone, Azithromycin, Cidofovir, Clarithromycin, Clindamycin, Cotrimoxazol, DDC, DDI, Dapson, Daunorubicin, Delavirdin, Doxorubicin, Efavirenz, Emtricitabin, Enfurvitide, Erythropoetin, Ethambutol, Filgrastim, Fluconazol, Fosamprenavir, Foscarnet, G-CSF, Ganciclovir, Indinavir, Interleukin-2, Interferon alpha, Isoniazid, Itraconazol, Lamivudin, Lenograstim, Lopinavir, Nelfinavir, Nevirapin, Pentamidin, Pyrimethaminm, Ribavirin, Rifabutin, Rifampicin, Ritonavir, Saquinavir, Stavudin, Sulfadiazin, T-20, Tenofovir, Tipranavir, Valganciclovir, Voriconazol, 3TC,
22. Calciumantagonisten
   Dihydropyridine (Nifedipin-Typ)
   Nifedipin, Nitrendipin, Felodipin, Amlodipin, ercanidipin, Nimodipin, Nicardipin, Lacidipin, Isradipin, Nisoldipin, Nilvadipin, Manidipin,
   Phenylalkylamine (Verapamil-Typ)
   Verapamil,Gallopamil, Fendilin
   Benzothiazepine (Diltiazem-Typ)
   Diltiazem

Pharmazeutische Wirkstoffe von besonderem Interesse sind Amphotericin B, Ciclosporin A, Aciclovir, Ritonavir, Paclitaxel, Taxane, Ketoconazol, Itraconazol, Ibuprofen, Naproxen, Omeprazol, Pantoprazol, Loratadin, Desloratadin, Loperamid, Daglutril.

Gemäß einer Ausführungsvariante wird die so erhaltene gefrorene Matrix in einem gekühlten Nichtlösemittel als äußerer Phase mittels herkömmlicher Rührmethoden bzw. Dispergiermethoden in gefrorenem Zustand dispergiert, so dass eine Mischung aus Eis und äußerer Phase entsteht.

Wenn es notwendig ist, können der äußeren Phase Tenside, Antiflokkulantien (z.B. Natriumcitrat) und polymere Stabilisatoren zugegeben werden.

Auf die so hergestellte Dispersion werden dann unmittelbar und vor dem Schmelzen der gefrorenen dispergierten Matrix mittlere oder hohe Scher- und/oder Kavitationskräfte angewendet. Mittlere Scherkräfte können durch Rotor-Stator-Rührsysteme (Leistungsdichte: 10⁶ / 10⁷ W/m³) oder alternative Geräte wie z.B. Zahnscheiben aufgebracht werden. Alternativ können Geräte mit höherer Leistungsdichte im Bereich von 10⁹ / 10¹³ W/m³ verwendet werden, mit deren Hilfe dann hohe Kräfte auf die Suspensionen aufgewendet werden können. Beispiele für solche Geräte sind Strahl-Homogenisatoren oder Kolben-Spalt-Homogenisatoren (z.B. Geräte der Serien Avestin, APV Gaulin, Niro Soavi) oder Ultraschallerzeuger der Firma Sonics.

Beispiel 1 zeigt die Ausführung der oben beschriebenen Variante der Erfindung unter Verwendung des Arzneistoffs Amphotericin B. Bereits nach 5 Homogenisationszyklen wurde eine Suspension mit einer mittleren Partikelgröße, die mit Hilfe der Photonenkorrelationsspektroskopie bestimmt wurde, von 143 nm erhalten werden. Nach sieben Tagen Lagerung nahm die mittlere Partikelgröße nur um 64 nm auf 207 nm zu, obwohl das verwendete Lösungsmittel Dimethylsulfoxid nicht aus dem System entfernt wurde. Dieses Beispiel zeigt, das bei Anwendung der erfinderischen Methode Nanosuspensionen mit im vergleich zu Hydrosolen deutlich verbesserten Lagerstabilitäten erzielt werden können.

Gemäß einer anderen Ausführungsvariante wird die nach dem Einfrieren erhaltene Matrix vor dem Dispergieren in der äußeren Phase in einem Gefriertrocknungsvorgang (Lyophilisation) schonend und langsam getrocknet, um das verwendete Lösungsmittel zu entfernen. Diese Ausführungsvariante bietet sich vor allem bei der Verwendung relativ toxischer Lösungsmittel an, oder wenn das verwendete Lösungsmittel mit der gewünschten äußeren Phase nicht mischbar ist. Nach dem Entfernen des Lösungsmittels wird die erhaltene Matrix analog zur ersten Ausführungsvariante weiterverarbeitet.

Beispiel 2 zeigt die Ausführung dieser Gefriertrocknung einbeziehenden Variante. Zum Einfrieren des Amphotericin B-Lösung wurde eine Gefriertruhe mit einer Temperatur von -20°C benutzt, was zu einem schnellen, aber nicht plötzlichem Einfrieren der Lösung führte. Nach 5 Homogenisationszyklen betrug die mittlere Partikelgröße bestimmt mit PCS 186 nm.

Im Gegensatz dazu wurde im Beispiel 3 die Amphotericin B-Lösung in flüssigem Stickstoff schockgefroren. Nach 5 Homogenisationszyklen betrug die mittlere Partikelgröße bestimmt mit PCS 62 nm. Man kann also feststellen, dass die Einfriergeschwindigkeit einen deutlichen Einfluss auf die später erreichbare Partikelgröße hat. Dies kann dadurch begründet werden, dass schnelleres Einfrieren zu kleineren Kristallen führt (Rudolf Voigt, Pharmazeutische Technologie für Studium und Beruf, Ullstein Mosby, Seite 59-60), die durch die anschließend aufgewendete Energie besser stabilisiert werden können.

In Beispiel 4 wurde der Arzneistoff Ciclosporin A entsprechend der ersten Ausführungsvariante prozessiert, wobei nach 15 Homogenisationszyklen eine mittlere Partikelgröße mit PCS von 630 nm ermittelt wurde.

Im Gegensatz dazu wurde in Beispiel 5 die zweite Ausführungsvariante des Patentes, also mit Lyophilisation, angewendet. Nach 15 Homogenisationszyklen wurden Partikel mit einem mittleren PCS-Durchmesser von 440 nm erhalten. Es zeigt sich also, dass die Anwendung der zweiten Ausführungsvariante im Allgemeinen zu einer kleineren Partikelgröße führt, allerdings muss dafür auch zusätzliche Energie in Form der Lyophilisation aufgebracht werden.

Um die hergestellten Partikel großtechnisch nutzen zu können, ist neben einer ausreichenden Stabilität in Form der Suspension auch eine Möglichkeit der Überführung in ein trocknes, lagerfähiges Produkt erforderlich.

Beispiel 6 zeigt die Lyophilisation der für Beispiel 3 hergestellten Nanosuspension. Die Lyophilisation führte zu einem lockeren, trockenen Produkt, aus dem durch Rekonstitution mit destilliertem Wasser wieder eine Nanosuspension mit annähernd derselben Partikelgröße, wie in Beispiel 3 erwähnt, erhalten werden konnte.

Beispiel 7 zeigt die Lyophilisation der für Bespiel 5 hergestellten Nanosuspension. Auch hier führte die Lyophilisation mit anschließender Rekonstitution zu einer vergleichbaren Partikelgröße.

Es kann also festgestellt werden, dass die hier vorgestellte Methode geeignet ist, in Wasser schwerlösliche Stoffe, insbesondere auch thermolabile und empfindliche Substanzen zu prozessieren. Mit wenigen Homogenisationszyklen bzw. durch die Aufwendung einer relativ geringen Leistungsdichte können Nanosuspensionen erhalten werden, deren mittlere Partikelgröße teilweise sogar weit unter 100 nm liegt. Außerdem besitzen die erzeugten Nanosuspensionen eine sehr gute Stabilität und können leicht in trockene Produkte bei gleich bleibend kleiner Partikelgröße überführt werden.

Die Partikelgrößenbestimmung wurde unter Nutzung der Laserdiffraktometrie (LD) und der Photonenkorrelationsspektroskopie (PCS) durchgeführt. Die Laserdiffraktometrie wurde mit einem Coulter LS 230 (Beckman-Coulter, USA) durchgeführt und liefert als Ergebnis einen volumenbezogene Partikelgrößenverteilung. Die zur Bestimmung herangezogenen Parameter waren die Durchmesser 50% (D 50%), 90% (D 90%) und 99% (D 99%). D 50% bedeutet z. B., dass 50% der Partikel bezogen auf deren Volumen einen Durchmesser unter dem angegebenen Wert besitzen. Die PCS-Analyse wurde mit einem Zetasizer 4 (Malvern Instruments, GB) durchgeführt. Die PCS ergibt einen mittleren Partikeldurchmesser (z-average) der Hauptpopulation und einen Polydisperitätsindex (PI) als Maß für die Breite der Partikelgrößenverteilung. Der PI für relativ enge Verteilungen liegt zwischen 0,1 - 0,2. Werte größer als 0,5 und mehr weisen auf eine sehr breite Partikelgrößenverteilung hin.

Ein schwerlöslicher Stoff im Sinne dieser Erfindung besitzt eine maximale Löslichkeit von 1%, bevorzugt kleiner 0,1% und insbesondere weniger als 0,01% im Dispersionsmedium (Angabe in Massenprozenten).

Die Erfindung ist dadurch charakterisiert, dass partikuläres Material in Nanometerbereich durch Aufwendung einer geringen Anzahl an Homogenisationszyklen bzw. durch eine relativ kurze Einwirkung von Scher- und Kavitationskräften erzielt werden kann. Bereits nach 1-5 Zyklen sind die Partikeldurchmesser normalerweise unter 1000 nm, sehr oft unter 400 nm und im Falle von weicheren Materialen unter 100 nm. Eine Erhöhung der Zyklenzahl ist nur im Falle von harten bis sehr harten Stoffen notwendig, maximal sind jedoch 15 bis 20 Zyklen erforderlich.

Die Herstellung von pharmazeutischen Wirkstoffen im Nanometerbereich ist für verschiedenste Applikationswege und Anwendungsbeispiele vorteilhaft und vorstellbar. In topischen Zubereitungen für Applikationen auf der Haut erhöhen nanokristalline Formen die Sättigungslöslichkeit, was zu einer verbesserten Penetration in die Haut führt. Bei der peroralen Verabreichung ist die Auflösungsgeschwindigkeit schwerlöslicher Wirkstoffe deutlich verbessert. Die erhöhte Sättigungslöslichkeit führt zu einem erhöhten Konzentrationsgradienten, was wiederum zu erhöhten Blutkonzentrationsspiegeln führt. Auch die parenterale Verabreichung über Injektionen und Infusionen ist möglich, wobei die sich schnell auflösenden Nanokristalle die Eigenschaften einer Lösung imitieren. Eine weitere Anwendung für Arzneistoffnanokristalle wären Opthalmika, z.B. könnte die Verabreichung am oder im Auge zu einer verlängerten Verweildauer des Wirkstoffs am Auge führen

Die hergestellten Nanopartikel könnten auch in andere Trägersysteme eingebracht werden und aufgrund ihrer Größe zu Vorteilen führen. Arzneistoffnanokristalle können durch die Verwendung geeigneter Tenside oder Stabilisatoren positiv geladen werden, was zu einer erhöhten Adhäsivität an der Haut und an Hautanhangsprodukten, wie z.B. Haaren, führt. Es sind auch Anwendungen in der Nahrungsmittelindustrie denkbar, schwerlösliche Hilfsstoffe könnten besser dispergiert und portioniert werden. Daneben sind nanokristalline Farbstoffe für die Verwendung in kosmetischen Produkten denkbar, aber auch von Farbpigmente für verschiedene andere Anwendungen. Nanokristallines Material kann auch in der Textilindustrie Anwendung finden.

Gemäß einer weiteren bevorzugten Ausführungsform beschreibt die vorliegende Erfindung auch einen mehrstufigen Prozess zur Herstellung von oberflächenmodifizierten Wirkstoffnanopartikeln bzw. Nanosuspensionen mittels Hochdruckhomogenisation von modifiziertem Wirkstoffmaterial in Anwesenheit von verschiedenen Polymeren bzw. Schutzkolloiden unter Ausschluss der Verwendung von Tensiden und/oder Emulgatoren. Auch die modifizierten Wirkstoffnanopartikel besitzen eine mittlere Partikelgröße von 10 nm bis unter.1000 nm. Als Nanosuspension vorliegend werden die modifizierten Wirkstoffnanopartikel ausschließlich durch die aufgebrachten Polyelektrolytmultischicht bzw. Polyelektrolytmultischichten stabilisiert und können entweder direkt als Nanosuspension angewendet werden oder zu trockenen Pulvern weiterverarbeitet werden.

Im allgemeinen Bedarf es zur Stabilisierung der auf diese Weise hergestellten kolloidalen Systeme einer Zugabe von Tensiden, Emulgatoren oder polymeren Stabilisatoren. Dabei werden die Tenside häufig im Verhältnis 1:1 bis 1:10 (Tensid zu Arzneistoff) eingesetzt. Durch die verwendeten Tenside können unerwünschte Effekte hervorgerufen werden, wie beispielsweise allergische Reaktionen.

Die vorliegende bevorzugte Ausführungsform ermöglicht jedoch die Herstellung von Nanosuspensionen unter Ausschluss von Tensiden durch Herstellung von oberflächenmodifizierten (polymerbeschichteten) Wirkstoffnanopartikeln.

Nach dem Stand der Technik wird eine Beschichtung von z.B. Mikro- und Nanokristallen (Templatpartikeln) erreicht, indem man eine Dispersion von Templatpartikeln (zu umhüllende Kristalle) oder feste Templatpartikel in einer salzhaltigen Flüssigphase, die die zur Beschichtung (Kapselbildung) erforderlichen Komponenten in gelöster Form enthält dispergiert und durch Präzipitation der Komponenten eine Kapselhülle gebildet wird (EP 01 305 109 B1).

Bisher wurde bei der Beschichtung von Templatpartikeln stets von Beschichtungsmaterial in gelöster Form (Polyelektrolytlösungen) ausgegangen. Die in gelöster Form vorliegenden Polyelektrolytketten können jedoch über sogenannte Brückenbildung das Auftreten einer starken, teilweise irreversiblen Aggregation der Templatpartikel hervorrufen, insbesondere wenn die Templatpartikeldispersion nicht mit Hilfe von Tensiden, Stabilisatoren oder anderen oberflächenaktiven Substanzen stabilisiert worden ist.

Der Überzug der Templatpartikel mit Polyelektrolytmultischichten erfolgt schrittweise, dass heisst die Templatpartikel werden mit mehreren (mindestens zwei) alternierenden Schichten von entgegengesetzt geladenen Polyelektrolyten überzogen. Nach jedem einzelnen Beschichtungsschritt sind die Templatpartikel in der Regel durch Filtration, Zentrifugation oder Dialyse von den überschüssigen Polymeren abzutrennen (wie in der US-A-6 833 192 oder der WO 2004/047977 A1 beschrieben), bevor die nächste Polyelektrolytschicht aufgetragen werden kann. Dadurch kommt es durch Filterrückstände einerseits und durch irreversible Aggregation und Agglomeration während der Zentrifugation andererseits zu relativ großen Verlusten an freibeweglichen Templatpartikeln.

Die vorliegende Ausführungsform ist daher ein kombiniertes Verfahren zur Herstellung von Wirkstoffnanopartikeln bei gleichzeitiger Oberflächenmodifikation zum Zwecke der Verringerung der Aggregations- und Agglomerationsneigung der hergestellten Partikel.

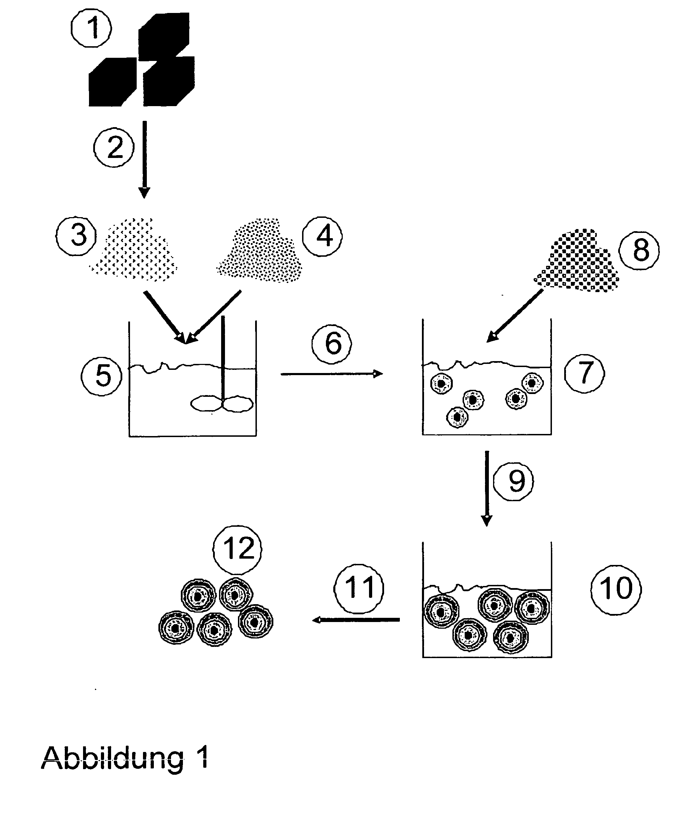

Auch diese Ausführungsform ist **dadurch gekennzeichnet, dass** die zu beschichtenden Wirkstoffnanopartikel im ersten Prozessschritt mit Hilfe der Hochdruckhomogenisation hergestellt werden. Dazu wird der schwer wasserlösliche bzw. wasserunlösliche Wirkstoff (siehe Abb. 1., Punkt 1) in einem geeigneten Lösungsmittel aufgelöst und die entstandene Lösung anschließend eingefroren (siehe Abb. 1, Punkt 2), so dass eine feste, gefrorene Matrix entsteht. Anschließend wird der gefrorenen Matrix entweder das Lösungsmittel mittels Lyophilisation entweder vollständig entzogen oder es wird mit der gefrorenen Matrix weitergearbeitet. Der modifizierte Wirkstoff (siehe Abb.1, Punkt 3) wird zusammen mit dem pulverförmigen Polymer 1 bzw. Schutzkolloid 1 (siehe Abb. 1, Punkt 4) in einer äußeren Phase mit Hilfe von geeigneten Mischern (z.B. Ultra-Turrax) dispergiert (siehe Abb. 1, Punkt 5). Wichtig ist, dass dabei nur das Polymer 1 bzw. Schutzkolloid 1 in der äußeren Phase löslich ist. Anschließend wird die Dispersion aus wasserlöslichem bzw. wasserunlöslichem Wirkstoff und festem Polymer 1 bzw. Schutzkolloid 1 mehreren Hochdruckhomogenisationszyklen unterworfen (siehe Abb. 1, Punkt 6), so dass eine metastabile Nanosuspension entsteht, wobei die Oberfläche der Wirkstoffnanopartikel mit Polymer 1 bzw. Schutzkolloid 1 besetzt ist (siehe Abb. 1, Punkt 7). Zu dieser metastabilen Nanosuspension gibt man anschließend das zu Polymer 1 bzw. Schutzkolloid 1 entgegengesetzt geladene Polymer 2 bzw. Schutzkolloid 2 (siehe Abb. 1, Punkt 8). Diese Mischung wird anschließend erneut homogenisiert (siehe Abb. 1, Punkt 9), wobei der Druck im Vergleich zu den ersten Homogenisationszyklen ((Abb. 1, Punkt 6) reduziert werden kann, da die Homogenisation nicht mehr der Partikelzerkleinerung dient. Die so hergestellten Nanopartikel (siehe Abb. 1, Punkt 10) besitzen eine entgegengesetzt gerichtete Oberflächenladung verglichen mit den Partikeln der metastabilen Nanosuspension (Abb. 1, Punkt 7). Zusätzlich ist die entstandene Nanosuspension nicht mehr metastabil, sondern besitzt eine ausgezeichnete physikalische Stabilität ohne Tendenz zur Partikelaggregation oder Agglomeration. Diese so hergestellten Nanosuspensionen können als Produkt verwendet oder weiterverarbeitet werden. Durch herkömmliche Trocknungsverfahren (siehe Abb. 1, Punkt 11), wie z.B. Sprühtrocknung, Lyophillisation oder einfache Filtration mit anschließender Trocknung des Filterkuchens entstehen nanokristalline Wirkstoffpulver (siehe Abb. 1, Punkt 12), die z.B. in Hartgelatinekapseln gefüllt werden oder zu Tabletten verpresst werden können.

Auch die mittels dieser Ausführungsform der Erfindung hergestellten oberflächenmodifizierten Partikel besitzen eine mittlere Partikelgröße von 10nm bis < 1000 nm, bevorzugt von 100 nm bis < 1000 nm, am meisten bevorzugt von 200 nm bis 500 nm.

Die zu verarbeitenden Wirkstoffe können auch hier aus verschiedensten Bereichen stammen, d.h. es können pharmazeutische Wirkstoffe, kosmetische Wirkstoffe, aber auch Zusatzstoffe für die Nahrungsmittelindustrie sowie Materialen für andere technische Bereiche verarbeitet werden, die bevorzugt als nanokristallines Material vorliegen sollen, wie z.B. Farbstoffe und Farbstoffpigmente für Farben und Lacke oder für kosmetische Anwendungen.

Eine Besonderheit dieser erfindungsgemäßen Ausführungsform ist, dass die in ihrer Oberflächeneigenschaft mit Hilfe von Polymeradsorption zu modifizierenden Wirkstoffnanopartikel direkt mittels Hochdruckhomogenisation bei gleichzeitig erfolgender Polymerbeschichtung im Prozess hergestellt werden. Darüber hinaus ist der Prozess der Partikelgrößenzerkleinerung aufgrund der Verwendung von speziell modifiziertem Ausgangsmaterial besonders effektiv, dass heißt, das zum Erreichen von Wirkstoffpartikelgrößen im Nanometerbereich (entsprechend Punkt 6, Abb. 1) häufig nur bis maximal 5 Homogenisationszyklen durchgeführt werden müssen, in besonderen Fällen nur 3 Homogenisationszyklen, speziell nur 1 Homogenisationszyklus.

Bei den Verfahren zur Partikelbeschichtung mit Polyelektrolytmultischichten nach dem Stand der Technik, erfolgt die Adsorption von Polyelektrolyten aufgrund von gegensätzlicher Ladung der verwendeten Polyelektrolyte, wobei zum Erreichen einer sogenannten Ladungsüberkompensation (es werden mehr Polyelektrolyte auf der Partikeloberfläche gebunden, als zum Ladungsausgleich erforderlich) ein Überschuss an Polyelektrolyten sowie ein bestimmter Salzgehalt erforderlich sind. Im Gegensatz dazu erfordert die erfinderische Methode keine Salzzugabe, da die Partikelbeschichtung aufgrund der aufgewendeten hohen Drücke eher aktiv erfolgt, das heisst die Polyelektrolyte werden unter Druck auf der Wirkstoffpartikeloberfläche abgeschieden. Es ist bekannt, dass die Zugabe von Salzen zu kolloidalen Systemen deren physikalische Stabilität aufgrund der Erniedrigung des Zetapotentials verringern kann. Aufgrund der Vermeidung von Salzzugabe ist die erzielbare physikalische Stabilität der nach der erfinderischen Methode hergestellten Suspensionen deutlich verbessert.

Als Polyelektrolyt geeignet sind sowohl niedermolekulare Polyelektrolyte bzw. Polyionen als auch makromolekulare Polyelektrolyte, beispielsweise Polyelektrolyte biologischer Herkunft.

Die Wirkstoffnanopartikel werden mit mindestens zwei Polyelektrolytschichten, das heißt mit mindestens einer positiven und einer negativen Polyelektrolytschicht (Schutzkolloidschicht) überzogen. Unter Polyelektrolyten versteht man allgemein Polymere mit ionisch dissoziierbaren Gruppen, die Bestandteil oder Substituent der Polymerkette sein können. Dabei ist die Zahl der dissoziierbaren Gruppen in Polyelektrolyten so gross, dass die Polymeren in der dissoziierten Form (auch Polyionen genannt) in der flüssigen Phase der Nanosuspension löslich sind. Je nach Art der dissoziierbaren Gruppen unterscheidet man bei Polyelektrolyten Polysäuren und Polybasen.

Polysäuren spalten bei der Dissoziation unter Bildung von Polyanionen Protonen ab. Beispiele für Polysäuren sind Polymethacrylate, Celluloseacetatphthalat (CAP), Hydroxypropylmethylcellulosephthalat (HPMCP), Hydroxypropylmethylcelluloseacetatsuccinat (HPMCAS), Polyacrylsäure, Alginsäure, Carboxymethylcellulose, Dextransulfat, Ligninsulfonsäure, Polyvinylsulfonsäure, Polyvinylphosphonsäure, Chondroitinsulfonsäure sowie deren Salze.

Verwendbare Biopolymere sind beispielsweise Gelatine A und Gelatine B, Chitosan und dessen Salze, Protaminsulfat, Hyaluronsäure, Polylysinsäure, Polymilchsäure, Carragenane, Pektine, Gummi Arabicum, Nucleinsäuren.

Polybasen enthalten protinierbare Gruppen, die in der Lage sind, Protonen, z. B. durch Reaktion mit Säuren unter Salzbildung, aufzunehmen. Beispiele für Polybasen mit ketten- bzw. seitenständigen dissoziierbaren Gruppen sind Polyethylenimin, Polyvinylamin und Polyvinylpyridin. Polybasen liegen nach deren Protonierung als Polykationen vor.

Ein besonderer Vorteil der Oberflächenmodifizierung gemäß dieser Ausführungsform besteht darin, dass zwischen den einzelnen Beschichtungsschritten keine Abtrennung von überschüssigen Polyelektrolyten mittels Abtrennverfahren, wie Zentrifugation, Filtration oder Dialyse, erfolgen muss. Zum einen können die erforderlichen Polymermengen in Vorversuchen bestimmt oder entsprechend berechnet werden, so dass genau die erforderliche Menge zugesetzt werden kann, ohne dass ein starker Polymerüberschuss erforderlich ist. Zum anderen stören überschüssig vorhandene Polymere den Herstellungsvorgang nicht. Es kann dann nur zur Bildung von wirkstofffreien Komplexen aus den gegensätzlich geladenen Polymeren oder Schutzkolloiden kommen, die aber keinen negativen Einfluss auf die Produkteigenschaften haben. Da auf Abtrennschritte während der Beschichtung der Wirkstoffnanopartikel verzichtet werden kann, ist das erfindungsgemäße Verfahren besonders geeignet, um als kontinuierliches Verfahren im industriellen Großmaßstab angewendet werden zu können.

Aufgrund der hohen Energie, die während der Hochdruckhomogenisation und der gleichzeitig erfolgenden Partikelbeschichtung ins System eingebracht wird, werden eventuell auftretende Aggregate aus Wirkstoffnanopartikeln sofort zerstört. Durch die Ausbildung eines stabilen, sehr hohen Zetapotentials durch Aufbringen des zweiten gegensätzlich geladenen Polymers (Abb. 1, Punkt 8) wird die Wirkstoffnanosuspension sehr gut stabilisiert und besitzt dann eine sehr gute physikalische Stabilität. Das Zetapotential (hierbei ist nur der Betrag entscheidend, und nicht das Vorzeichen der Ladung) der nach dem erfindungsgemäßen Verfahren hergestellten Nanosuspension, gemessen in Wasser mit einer Leitfähigkeit im Bereich von 50µS bei pH Werten zwischen 4 bis 7, liegt im Bereich von 5 mV bis 100 mV, bevorzugt im Bereich von 20 mV bis 80 mV, besonders bevorzugt im Bereich von 30 mV bis 60 mV.

Aufgrund der hohen Oberflächenladung und der stabilen Haftung der Polyelektrolytschicht auf den Wirkstoffnanokristallen besitzen sowohl die Nanosuspensionen selbst als auch die durch Trocknung erhaltenen Pulver eine ausgezeichnete physikalische Stabilität unter Elektrolyteinfluss.

Ein weiterer Vorteil des erfindungsgemäßen Verfahrens ist die Möglichkeit des vollständigen Ausschlusses von Tensiden während des Herstellungsprozesses. Im Gegensatz zum bisherigen Stand der Technik konnte gezeigt werden, dass kolloidale Wirkstoffsuspensionen auch unter vollständigen Ausschluss von Tensiden mittels Hochdruckhomogenisation herstellbar sind (siehe Beispiele 8 bis 12). Das ist insbesondere von Vorteil, wenn die nach dem erfindungsgemäßen Verfahren hergestellten Nanosuspensionen als Arzneimittel angewendet werden sollen, oder zu Arzneimitteln weiterverarbeitet werden sollen. Der Ausschluss von Tensiden ist insbesondere für die Herstellung von Wirkstoffnanosuspensionen zu parenteralen Applikation von besonderer Bedeutung.

### Beispiel 1

400 mg Amphotericin B wurden in 10 mL Dimethylsulfoxid aufgelöst. Auf diese Lösung wurde flüssiger Stickstoff gegeben, was zu einem sofortigen Einfrieren der Arzneistofflösung führte. Nachdem der flüssige Stickstoff abgedampft war, wurde die dabei erhaltene poröse Matrix bestehend aus gefrorenem Dimethylsulfoxid und Amphotericin B mit Hilfe eines Ultra-Turrax (Janke & Kunkel, Germany) 5 Sekunden lang bei 9500 Umdrehungen pro Minute in 30 g einer wässrigen 1,1% Natriumcholat-Lösung (m/m) dispergiert und sofort in einem Hochdruckhomogenisator MicronLab 40 (APV Gaulin, Deutschland) mit 1500 bar bei einer Gerätetemperatur von 10°C homogenisiert. Nach 5 Homogenisationszyklen betrug der mittlere Partikeldurchmesser, vermessen mit Hilfe der Photonenkorrelationsspektroskopie (PCS), 143 nm bei einem Polydispersitätsindex (PI) von 0,252. Die mit Hilfe der Laserdiffraktometrie (LD) bestimmten Volumenverteilungen betrugen D50% 70 nm, D90% 209 nm und D99% 279 nm. Nach einer Lagerungszeit von 7 Tagen bei Raumtem-peratur (RT) betrug der mittlere Partikeldurchmesser vermessen mit PCS 207,1 nm und die Volumenverteilungen D50% 136,0 nm, D 90% 193,0 nm und D99% 452,0 nm.

### Beispiel 2

400 mg Amphotericin B wurden in 10 mL Dimethylsulfoxid aufgelöst. Diese Lösung wurde dann bei -20°C eingefroren und anschließend in einer Lyophilisationsapparatur Christ alpha I-5 (Christ-Apparatebau, Osterode, Germany) lyophilisiert. Die dabei erhaltene poröse Matrix wurde mit Hilfe eines Ultra-Turrax (Janke & Kunkel, Germany) 10 Sekunden lang bei 9500 Umdrehungen pro Minute in 39,6 g einer wässrigen 1,1% Natriumcholat-Lösung (m/m) dispergiert und sofort in einem Hochdruckhomogenisator MicronLab 40 (APV Gaulin, Deutschland) mit 1500 bar bei einer Gerätetemperatur von 0°C homogenisiert. Nach 5 Homogenisationszyklen betrug der mittlere Partikeldurchmesser, vermessen mit PCS 186 nm bei einem PI von 0,411. Die Volumenverteilungen betrugen D50% 78 nm, D90% 238 nm und D99% 446 nm.

### Beispiel 3

400 mg Amphotericin B wurden in 10 mL Dimethylsulfoxid aufgelöst. Auf diese Lösung wurde dann flüssiger Stickstoff gegeben, was zu einem sofortigen Einfrieren der Arzneistofflösung führte. Die gefrorene Lösung wurde anschließend in einer Lyophilisationsapparatur Christ alpha I-5 (Christ-Apparatebau, Osterode, Germany) lyophilisiert. Die dabei erhaltene poröse Matrix wurde mit Hilfe eines Ultra-Turrax (Janke & Kunkel, Germany) 10 Sekunden lang bei 9500 Umdrehungen pro Minute in 39,6 g einer wässrigen 1,1% Natriumcholat-Lösung (m/m) dispergiert und sofort in einem Hochdruckhomogenisator MicronLab 40 (APV Gaulin, Deutschland) mit 1500 bar bei einer Gerätetemperatur von 0°C homogenisiert. Nach 5 Homogenisationszyklen betrug der mittlere Partikeldurchmesser, vermessen mit PCS 62 nm bei einem PI von 0,555. Die Volumenverteilungen betrugen D50% 60 nm, D90% 79 nm und D99% 98 nm.

### Beispiel 4

400 mg Ciclosporin A wurden in 10 mL Ethanol aufgelöst. Auf diese Lösung wurde flüssiger Stickstoff gegeben, was zu einem sofortigen Einfrieren der Arzneistofflösung führte. Nachdem der flüssige Stickstoff abgedampft war, wurde die dabei erhaltene poröse Matrix aus gefrorenem Ethanol und Ciclosporin in 30 g einer wässrigen 1,1% Poloxamer 188-Lösung (m/m) mit Hilfe eines Spatels grob dispergiert und sofort in einem Hochdruckhomogenisator MicronLab 40 (APV Gaulin, Deutschland) mit 1500 bar bei einer Gerätetemperatur von 0°C homogenisiert. Nach 15 Homogenisationszyklen betrug der mittlere Partikeldurchmesser, vermessen mit PCS 630 nm bei einem PI von 0,302. Die Volumenverteilungen betrugen D50% 794 nm, D90% 1717 nm und D99% 3857 nm.

### Beispiel 5

400 mg Ciclosporin A wurden in einer Mischung aus 10 mL Ethanol und 10 mL Dimethylsulfoxid aufgelöst. Auf diese Lösung wurde flüssiger Stickstoff gegeben, was zu einem sofortigen Einfrieren der Arzneistofflösung führte. Die gefrorene Lösung wurde anschließend in einer Lyophilisationsapparatur Christ alpha I-5 (Christ-Apparatebau, Osterode, Germany) lyophilisiert. Die dabei erhaltene poröse Matrix wurde mit Hilfe eines Ultra-Turrax (Janke & Kunke, Germany) 10 Sekunden lang bei 9500 Umdrehungen pro Minute in 39,6 g einer wässrigen 1,1% Poloxamer 188-Lösung (m/m) dispergiert und sofort in einem Hochdruckhomogenisator MicronLab 40 (APV Gaulin, Deutschland) mit 1500 bar bei einer Gerätetemperatur von 0°C homogenisiert. Nach 15 Homogenisationszyklen betrug der mittlere Partikeldurchmesser, vermessen mit PCS 440 nm bei einem PI von 0,264. Die Volumenverteilungen betrugen D50% 405 nm, D90% 1790 nm und D99% 2321 nm.

### Beispiel 6

1 mL der bei Beispiel 3 erhaltenen Suspension wurde mit 10 mg Fructose versetzt. Diese Mischung wurde unverzüglich in flüssigem Stickstoff eingefroren. Die gefrorene Mischung wurde anschließend in einer Lyophilisationsapparatur Christ alpha I-5 (Christ-Apparatebau, Osterode, Germany) lyophilisiert. Die dabei erhaltene poröse Matrix wurde in destilliertem Wasser resuspendiert. Der mittlere Partikeldurchmesser, vermessen mit PCS, betrug 61 nm bei einem PI von 0,455.

### Beispiel 7

1 mL der bei Beispiel 3 erhaltenen Suspension wurde mit 10 mg Fructose versetzt. Diese Mischung wurde unverzüglich in flüssigem Stickstoff eingefroren. Die gefrorene Mischung wurde anschließend in einer Lyophilisationsapparatur Christ alpha I-5 (Christ-Apparatebau, Osterode, Germany) lyophilisiert. Die dabei erhaltene poröse Matrix wurde in destilliertem Wasser resuspendiert. Der mittlere Partikeldurchmesser, vermessen mit PCS, betrug 574 nm bei einem PI von 0,444.

### Beispiel 8:

4,0 g mikronisiertes Ibuprofen wurden in 36,0 mL angesäuertem Wasser (pH 2,5) unter Zusatz von 36,0 mg festen pulverförmigen Eudragit E (kationisches Schutzkolloid 1) mit Hilfe eines Ultra-Turrax (Jahnke & Kunkel, Germany) 5 Sekunden lang bei 9500 Umdrehungen pro Minute dispergiert. Die erhaltene Dispersion wurde in einem Hochdruckhomogenisator Micron Lab 40 (APV Systems, Deutschland) mit 1500 bar bei Raumtemperatur homogenisiert. Nach 5 Homogenisationszyklen wurde von der erhaltenen metastabilen Rohsuspension das Zetapotential bestimmt. Der Wert für das Zetapotential (gemessen in Wasser mit einem pH-Wert eingestellt auf 3,8 und einer Leitfähigkeit eingestellt auf 50 µS) betrug: 75,2 mV. Nach Zusatz von 400 mg fester, pulverförmiger Polyacrylsäure (anionisches Schutzkolloid 2) (pH Messung/Einstellung auf pH 3,8)wurde die metastabile Rohsuspension erneut für 5 Zyklen in einem Hochdruckhomogenisator Micron Lab 40 (APV Systems, Deutschland) mit 1500 bar bei Raumtemperatur homogenisiert. Als Endprodukt wurde eine physikalisch stabile, homogene Suspension erhalten, welche weder eine Tendenz zur Partikelaggregation noch zur Agglomeration aufwies, was mit Hilfe eines Lichtmikroskopes bestätigt werden konnte. Anschließend wurde erneut das Zetapotential der Suspension (gemessen in Wasser mit einem pH-Wert eingestellt auf 3,8 und einer Leitfähigkeit eingestellt auf 50µS) bestimmt, dessen Wert: -22,7 mV betrug.

### Beispiel 9:

4,0 g Ibuprofen wurden in 10,0 mL Ethanol gelöst. Auf diese Lösung wurde flüssiger Stickstoff gegeben, was zu einem sofortigen Einfrieren der Arzneistofflösung führte. Nachdem der flüssige Stickstoff abgedampft war, wurde die dabei erhaltene poröse Matrix bestehend aus gefrorenem Ethanol und Ibuprofen mit Hilfe eines Ultra-Turrax (Jahnke & Kunkel, Germany) 5 Sekunden lang bei 9500 Umdrehungen pro Minute in 36,0 mL angesäuertem Wasser (pH 2,5) unter Zusatz von 36,0 mg festem pulverförmigen Eudragit E (kationisches Schutzkolloid 1) dispergiert und sofort in einem Hochdruckhomogenisator Micron Lab 40 (APV Systems, Deutschland) mit 1500 bar bei Raumtemperatur homogenisiert. Nach 5 Homogenisationszyklen wurde von der erhaltenen metastabilen Rohsuspension das Zetapotential bestimmt. Der Wert für das Zetapotential (gemessen in Wasser mit einem pH-Wert eingestellt auf 3,8 und einer Leitfähigkeit eingestellt auf 50 µS) betrug: 41,6 mV. Nach Zusatz von 400mg fester, pulverförmiger Polyacrylsäure (Carbopol 980) (anionisches Schutzkolloid 2) (pH Messung/Einstellung auf pH 3,8) wurde die metastabilen Rohsuspension erneut für 5 Zyklen in einem Hochdruckhomogenisator Micron Lab 40 (APV Systems, Deutschland) mit 1500 bar bei Raumtemperatur homogenisiert. Als Endprodukt wurde eine physikalisch stabile, homogene Suspension erhalten, welche weder eine Tendenz zur Partikelaggregation noch zur Agglomeration aufwies, was wiederum mit Hilfe eines Lichtmikroskopes bestätigt werden konnte. Anschließend wurde erneut das Zetapotential der Suspension bestimmt (gemessen in Wasser mit einem pH-Wert eingestellt auf 3,8 und einer Leitfähigkeit eingestellt auf 50 µS), dessen Wert: -31,3 mV betrug.

### Beispiel 10:

4,0 g Ibuprofen wurden in 10,0 mL Aceton gelöst. Auf diese Lösung wurde flüssiger Stickstoff gegeben, was zu einem sofortigen Einfrieren der Arzneistofflösung führte. Nachdem der flüssige Stickstoff abgedampft war, wurde die dabei erhaltene poröse Matrix bestehend aus gefrorenem Aceton und Ibuprofen mit Hilfe eines Ultra-Turrax (Jahnke & Kunkel, Germany) 5 Sekunden lang bei 9500 Umdrehungen pro Minute in 36,0 mL angesäuertem Wasser (pH 2,5) unter Zusatz von 36 mg festen, pulverförmigen Eudragit E (kationisches Schutzkolloid 1) dispergiert und sofort in einem Hochdruckhomogenisator Micron Lab 40 (APV Systems, Deutschland) mit 1500 bar bei Raumtemperatur homogenisiert. Nach 5 Homogenisationszyklen wurde von der erhaltenen metastabilen Rohsuspension das Zetapotential bestimmt. Der Wert für das Zetapotential (gemessen in Wasser mit einem pH-Wert eingestellt auf 3,8 und einer Leitfähigkeit eingestellt auf 50µS) betrug: 6,2 mV. Nach Zusatz von 400 mg fester, pulverförmiger Polyacrylsäure (Carbopol 980) (anionisches Schutzkolloid 2) (pH Messung/Einstellung auf pH 3,8) wurde die metastabilen Rohsuspension erneut für 5 Zyklen in einem Hochdruckhomogenisator Micron Lab 40 (APV Systems, Deutschland) mit 1500 bar bei Raumtemperatur homogenisiert. Als Endprodukt wurde eine physikalisch stabile, homogene Suspension erhalten, welche weder eine Tendenz zur Partikelaggregation noch zur Agglomeration aufwies, was wiederum mit Hilfe eines Lichtmikroskopes bestätigt werden konnte. Anschließend wurde erneut das Zetapotential der Suspension bestimmt(gemessen in Wasser mit einem pH-Wert eingestellt auf 3,8 und einer Leitfähigkeit eingestellt auf 50 µS), dessen Wert: -31,9 mV betrug.

### Beispiel 11:

0,4 g Hydrocortisonacetat wurden in 10mL Dimethylsulfoxid aufgelöst. Auf diese Lösung wurde dann flüssiger Stickstoff gegeben, was zu einem sofortigen Einfrieren der Arzneistofflösung führte. Die gefrorene Lösung wurde anschließend in einer Lyophilisationsapparatur Christ alpha I-5 (Christ-Apparatebau, Osterode, Germany) 48 Stunden lang lyophilisiert. Die dabei erhaltene poröse Matrix wurde mit 200 mg festem, pulverförmigen Chitosanhydrochlorid (kationisches Schutzkolloid 1) versetzt und mit Hilfe eines Ultra-Turrax (Janke & Kunkel, Germany) 5 Sekunden lang bei 9500 Umdrehungen pro Minute in 39,2 g Wasser dispergiert und sofort in einem Hochdruckhomogenisator Micron Lab 40 (APV Systems, Deutschland) mit 1500 bar bei Raumtemperatur homogenisiert Die nach 5 Homogenisationszyklen erhaltene metastabile Rohsuspension wurde mikroskopische betrachtet und es wurden mikroskopische Aufnahmen gemacht. Der Wert für das Zetapotential (gemessen in Wasser mit einem pH-Wert eingestellt auf 6,5 und einer Leitfähigkeit eingestellt auf 50µS) betrug: 47,8 mV. Nach Zusatz von 400 mg fester, pulverförmiger Gelatine B (anionisches Schutzkolloid 2) (pH Messung/Einstellung auf pH 7,0) wurde die metastabile Rohsuspension erneut für 5 Zyklen in einem Hochdruckhomogenisator Micron Lab 40 (APV Systems, Deutschland) mit 1500 bar bei Raumtemperatur homogenisiert. Als Endprodukt wurde eine physikalisch stabile, homogene Suspension erhalten, welche weder eine Tendenz zur Partikelaggregation noch zur Agglomeration aufwies, was mit Hilfe eines Lichtmikroskopes bestätigt werden konnte. Anschließend wurde erneut das Zetapotential der Suspension bestimmt (gemessen in Wasser mit einem pH-Wert eingestellt auf 6,5 und einer Leitfähigkeit eingestellt auf 50 µS), dessen Wert: -16,9 mV betrug.

### Beispiel 12:

0,4 g Hydrocortisonacetat wurden in 10 mL Dimethylsulfoxid aufgelöst. Auf diese Lösung wurde dann flüssiger Stickstoff gegeben, was zu einem sofortigen Einfrieren der Arzneistofflösung führte. Die gefrorene Lösung wurde anschließend in einer Lyophilisationsapparatur Christ alpha I-5 (Christ-Apparatebau, Osterode, Germany) 48 Stunden lang lyophilisiert. Die dabei erhaltene poröse Matrix wurde mit 200 mg festem, pulverförmigen Chitosanhydrochlorid (kationisches Schutzkolloid 1) versetzt und mit Hilfe eines Ultra-Turrax (Janke & Kunkel, Germany) 5 Sekunden lang bei 9500 Umdrehungen pro Minute in 39,2 g Wasser dispergiert und sofort in einem Hochdruckhomogenisator Micron Lab 40 (APV Systems, Deutschland) mit 1500 bar bei Raumtemperatur homogenisiert. Die nach 5 Homogenisationszyklen erhaltene metastabile Rohsuspension wurde mikroskopische betrachtet und es wurden mikroskopische Aufnahmen gemacht. Der Wert für das Zetapotential (gemessen in Wasser mit einem pH-Wert eingestellt auf 6,5 und einer Leitfähigkeit eingestellt auf 50 µS) betrug: 47,8 mV. Nach Zusatz von 400 mg fester, pulverförmiger Polyacrylsäure (Carbopol 980) (anionisches Schutzkolloid 2) wurde die metastabile Rohsuspension erneut für 5 Zyklen in einem Hochdruckhomogenisator Micron Lab 40 (APV Systems, Deutschland) mit 1500 bar bei Raumtemperatur homogenisiert. Als Endprodukt wurde eine physikalisch stabile, homogene Suspension erhalten, welche weder eine Tendenz zur Partikelaggregation noch zur Agglomeration aufwies. Anschließend wurde erneut das Zetapotential der Suspension bestimmt (gemessen in Wasser mit einem pH-Wert eingestellt auf 6,5 und einer Leitfähigkeit eingestellt auf 50 µS), dessen Wert: -34,2 mV betrug.

Der mittlere Partikeldurchmesser, vermessen mit Hilfe der Photonenkorrelationsspektroskopie (PCS), betrug 1025,4nm bei einem Polydispersitätsindex (PI) von 0,294. Die mit Hilfe der Laserdiffraktometrie (D) bestimmten Volumenverteilungen betrugen D50% 414 nm, D90% 1977 nm und D95% 2926 nm.

## Patentansprüche

1. Verfahren zur schonenden Herstellung von hochfeinen Partikelsuspensionen **dadurch gekennzeichnet, dass**
a) ein in Wasser unlöslicher oder in Wasser schwerlöslicher Feststoff in einem geeigneten Lösungsmittel gelöst wird,
b) die Lösung aus a) anschließend unter Bildung einer festen Matrix eingefroren wird,
c) gegebenenfalls der in b) gebildeten festen Matrix in gefrorenem Zustand das Lösungsmittel durch Trocknung, insbesondere Lyophilisation, entzogen wird,
d) die in b) gebildete feste Matrix, die ggf. gemäß c) getrocknete, insbesondere lyophilisiert, worden ist, in einem Dispergiermittel in gefrorenem Zustand dispergiert wird, und
e) anschließend auf die in d) hergestellte Dispersion vor dem Schmelzen der gefrorenen, dispergierten, festen Martix mittlere bis hohe Kräfte aufgebracht werden, so dass eine Partikelsuspension entsteht, deren mittlere Partikelgröße, bestimmt mittels Photonenkorrelationsspektroskopie (PCS), unter 1000 nm, insbesondere im Bereich von 50 bis < 1000 nm, bevorzugt unter 800 nm, vorzugsweise im Bereich von 50 bis 600 nm, und insbesondere unter 400, bevorzugt im Bereich von 50 bis 200 nm, und speziell unter 100 nm liegt.

2. Verfahren nach Anspruch 1, **dadurch gekennzeichnet, dass** der zu lösende Feststoff ein Arzneimittelwirkstoff, ein kosmetischer Wirkstoff, ein Zusatzstoff für Nahrungsmittel, ein Farbstoff oder ein Pigment ist.

3. Verfahren nach einem der Ansprüche 1 oder 2, **dadurch gekennzeichnet, dass** die mittleren bis hohen Kräfte Scher-, Kavitations-, Mahl- und/oder Ultraschallkräfte sind, die insbesondere duch Hochdruckhomogenisatoren, Jet-Stream-Geräte, Rotor-Stator-Kolloidmühlen, Kugelmühlen, Hochscherungsmischer oder Ultraschallapparaturen aufgebracht werden, wobei das eingesetzte Gerät vorzugsweise mit einer Leistungsdichte von 10⁶ bis 10¹³/m³ arbeitet, insbesondere im Bereich von 10⁹ bis 10¹³/m³.

4. Verfahren nach einem der Ansprüche 1 bis 3, **dadurch gekennzeichnet, dass** die für die Auflösung des in Wasser unlöslichen oder in Wasser schwerlöslichen Feststoffs eingesetzten Lösungsmittel, hydrophile Flüssigkeiten, insbesondere Alkohole, bevorzugt Methanol, Ethanol und Isopropanol, Mischungen von Wasser mit mit Wasser vollständig oder teilweise mischbaren Flüssigkeiten oder hydrophilen Flüssigkeiten, insbesondere Alkoholen, bevorzugt Methanol, Ethanol oder Isopropanol oder andere organischen Lösungsmitteln, oder mit Wasser nicht mischbare Flüssigkeiten, insbesondere Chloroform oder Dichlormethan sind, wobei bevorzugte Lösungsmittel N-Methyl-2-pyrrolidinon, 2-Pyrrolidon, Dimethylacetamid, Ethanol, Aceton, Chloroform, Dichlormethan, Dimethylsulfoxid, N-Propanol, Glycerol, Ethylenglycol, Dimethylformamid, Dimethylacetamid oder Säuren und Basen, insbesondere Chlorwasserstoffsäure, Schwefelsäure, Essigsäure, Ameisensäure, Triethanolamin, Pyridin, Ammoniak sind, wobei gegebenenfalls eine Mischung aus zwei oder mehr derselben eingesetzt wird.

5. Verfahren nach einem der Ansprüche 1 bis 4, **dadurch gekennzeichnet, dass** die in a) hergestellte Feststofflösung noch einen oder mehrere weitere Hilfsstoffe und dispersionsstabilisierende Substanzen enthält, insbesondere Tenside, Stabilisatoren vom Typ der Antiflokkulantien und Polymere, sowie inerte Füllstoffe, wobei die Konzentrationen pro Komponente, bezogen auf das Gewicht, bevorzugt im Bereich von 1-90%, insbesondere von 1-20% und bevorzugt unterhalb von 10% liegen, idealerweise unterhalb von 0,01-5% liegen.

6. Verfahren nach Anspruch 5, **dadurch gekennzeichnet, dass** die stabilisierenden Substanzen Verbindungen aus der Reihe der Poloxamere, Poloxamine, ethoxylierten Mono- und Diglyceride, ethoxylierten Lipide und Lipoide, ethoxylierten Fettalkohole und Alkylphenole, ethoxylierten Fettsäureester, Polyglycerinether und -ester, Lecithine, Ester und Ether von Zuckern oder Zuckeralkoholen mit Fettsäuren oder Fettalkoholen, Phospholipide und Sphingolipide, Sterine, deren Ester oder Ether sowie deren Mischungen dieser Verbindungen umfassen.

7. Verfahren nach Anspruch 5, **dadurch gekennzeichnet, dass** die stabilisierenden Substanzen Eilecithin, Sojalecithin oder hydriertes Lecithin, deren Mischungen oder Mischungen aus einem oder beiden Lecithinen mit einer oder mehreren Phopholipidkomponenten, Cholesterin, Cholesterinpalmitat, Stigmasterin oder andere Sterine umfassen.

8. Verfahren nach Anspruch 5, **dadurch gekennzeichnet, dass** die Stabilisatoren Diacetylphosphat, Phosphatidylglycerol, gesättigte oder ungesättigte Fettsäuren, Natriumcholat, Peptisatoren oder Aminosäuren umfassen.

9. Verfahren nach einem der Ansprüche 5 bis 8, **dadurch gekennzeichnet, dass** die Feststofflösung einen oder mehrere viskositätserhöhende Stoffe, insbesondere Celluloseether und -ester, Polyvinylderivate, Alginate, Xanthane, Pektine, Polyacrylate, Poloxamere und Poloxamine, Polyvinlyalkohol, Polyvinylpyrrolidon enthält.

10. Verfahren nach einem der Ansprüche 5 bis 9, **dadurch gekennzeichnet, dass** die Feststofflösung außerdem einen oder mehrere weitere Hilfsstoffe, insbesondere Zucker oder Zuckeralkohole, bevorzugt Glucose, Mannose, Trehalose, Mannit und Sorbit, Fructose, Natriumcitrat, Natriumhydrogenphosphat, Natriumdihydrogenphosphat, Natriumchlorid, Kaliumchlorid, Glycerin, Farbstoffe oder Pigment enthält.

11. Verfahren nach einem der Ansprüche 1 bis 10, **dadurch gekennzeichnet, dass** für den Einfriervorgang in b) Verfahren verwendet werden, durch die ein vollständiges Durchfrieren des gerade einzufrierenden Teils der hergestellten Lösung innerhalb von weniger als 60 Sekunden, bevorzugt weniger als 30 Sekunden, besonders bevorzugt weniger als 10 Sekunden, speziell weniger als 1 Sekunde erfolgt.

12. Verfahren nach einem der Ansprüche 1 bis 12, **dadurch gekennzeichnet, dass** der durch Temperaturentzug in b) entstehenden festen gefrorenen Matrix vor der Dispergierung in dem die äußere Phase bildenden Dispergiermittel, durch Lyophilisation das Lösungsmittel entzogen wird, insbesondere bei der Verwendung von für die direkte Anwendung an Mensch und Tier ungeeigneten Lösungsmitteln.

13. Verfahren nach einem der Ansprüche 1 bis 12 **dadurch gekennzeichnet, dass** die Trocknung schonend und langsam über mehrere Stunden, bevorzugt weniger als 168 Stunden, besonders bevorzugt weniger als 72 Stunden, insbesondere weniger als 24 Stunden, in speziellen Fällen weniger als 12 Stunden, in einer geeigneten Lyophilisationsapparatur bei erniedrigten Drücken, bevorzugt bei 0,5 mbar, besonders bevorzugt bei 0,1 mbar, insbesondere bei 0,05 mbar, und bei Temperaturen von bevorzugt unter 20°C, insbesondere unter 0°C und speziell unter -20°C erfolgt.

14. Verfahren nach einem der Ansprüche 1 bis 13, **dadurch gekennzeichnet, dass** die nach dem Entzug des oder der Lösungsmittel erhaltene feste Matrix den Feststoff in kristalliner, teilkristalliner oder amorpher Form enthält und einen Restgehalt an Lösungsmittel, bezogen auf das Gewicht, von weniger als 50 Prozent, bevorzugt weniger als 10 Prozent, besonders bevorzugt weniger als 5 Prozent, speziell weniger als 1 Prozent enthält.

15. Verfahren nach einem der Ansprüche 1 bis 14, **dadurch gekennzeichnet, dass** die nach dem Entzug des Lösungsmittels erhaltene feste Matrix in einem Dispersionsmedium dispergiert wird, insbesondere durch Rühren mit Blattrührern, Rotor-Stator-Systemen oder statischen Mischern, so dass eine Dispersion erhalten wird.

16. Verfahren nach Anspruch 15, **dadurch gekennzeichnet, dass** als Dispersionsmedium Wasser, Mischungen aus Wasser und mit Wasser mischbaren Flüssigkeiten, nicht-wäßrige Medien oder organische Lösungsmittel oder lipophile Flüssigkeiten, insbesondere Öle und fette Öle, eingesetzt werden, in denen der Feststoff schwer- bzw. unlöslich ist.

17. Verfahren nach einem der Ansprüche 1 bis 16, **dadurch gekennzeichnet, dass** die in d) hergestellte Dispersion noch einen oder mehrere weitere Hilfsstoffe und eine oder mehrere dispersionsstabilisierende Substanzen enthalten kann, insbesondere Tenside, Stabilisatoren vom Typ der Antiflokkulantien und Polymere, sowie inerte Füllstoffe, wobei die Konzentrationen pro Komponente, bezogen auf das Gewicht, bevorzugt im Bereich von 1-90%, insbesondere von 1-20% und bevorzugt unterhalb von 10% liegen, idealerweise unterhalb von 0,01-5% liegen.

18. Verfahren nach Anspruch 17, **dadurch gekennzeichnet, dass** die dispersionsstabilisierenden Substanzen Verbindungen aus der Reihe der Poloxamere, Poloxamine, ethoxylierten Mono- und,Diglyceride, ethoxylierten Lipide und Lipoide, ethoxylierten Fettalkohole und Alkylphenole, ethoxylierten Fettsäureester, Polyglycerinether und -ester, Lecithine, Ester und Ether von Zuckern oder Zuckeralkoholen mit Fettsäuren oder Fettalkoholen, Phospholipide und Sphingolipide, Sterine, deren Ester oder Ether sowie deren Mischungen dieser Verbindungen umfassen.

19. Verfahren nach Anspruch 17, **dadurch gekennzeichnet, dass** die dispersionsstabilisierenden Substanzen Eilecithin, Sojalecithin oder hydriertes Lecithin, deren Mischungen oder Mischungen aus einem oder beiden Lecithinen mit einer oder mehreren Phopholipidkomponenten, Cholesterin, Cholesterinpalmitat, Stigmasterin oder andere Sterine umfassen.

20. Verfahren nach Anspruch 17, **dadurch gekennzeichnet, dass** die Stabilisatoren Dicetylphosphat, Phosphatidylglycerol, gesättigte oder ungesättigte Fettsäuren, Natriumcholat, Peptisatoren oder Aminosäuren umfassen.

21. Verfahren nach einem der Ansprüche 1 bis 20, **dadurch gekennzeichnet, dass** in der Dispersion viskositätserhöhende Stoffe, insbesondere Celluloseether und -ester, Polyvinylderivate, Alginate, Xanthane, Pektine, Polyacrylate, Poloxamere und Poloxamine, Polyvinlyalkohol, Polyvinylpyrrolidon enthalten sind.

22. Verfahren nach einem der Ansprüche 1 bis 21, **dadurch gekennzeichnet, dass** die Dispersion außerdem Hilfsstoffe wie Zucker oder Zuckeralkohole, insbesondere Glucose, Mannose, Trehalose, Mannit und Sorbit, Fructose oder Hilfsstoffe wie Natriumcitrat, Natriumhydrogenphosphat, Natriumdihydrogenphosphat, Natriumchlorid, Kaliumchlorid, Calciumchlorid, Glycerin enthält.

23. Verfahren nach einem der Ansprüche 1 bis 22, **dadurch gekennzeichnet, dass** die aufgewendete Energie durch einen Hochdruckprozess aufgewendet wird, wobei insbesondere Homogenisatoren vom Kolben-Spalt-Typ (z.B. APV Gaulin, NiroSoavi, Avestin), vom Jet-Stream-Typ (z.B. Microfluidizer) oder eine French Press (SLM Instruments, Urbana, USA) eingesetzt werden.

24. Verfahren nach einem der Ansprüche 1 bis 23 **dadurch gekennzeichnet, dass** bei Verwendung von Hochdruckhomogenisatoren der Homogenisationsdruck oberhalb 100 bar liegt, bevorzugt bei oder oberhalb 500 bar, insbesondere bei oder oberhalb 1500 bar und am günstigsten bei oder oberhalb 2000 bar.

25. Verfahren nach einem der Ansprüche 1 bis 24, **dadurch gekennzeichnet, dass** bei Verzicht auf die Trocknung der gefrorenen Matrix diese im gefrorenen Zustand in einer äußeren Phase dispergiert wird und die Kräfte auf die dispergierte noch gefrorene Matrix einwirken, so dass ein Schmelzen der gefrorenen Matrix und ein damit verbundenes Freiwerden der ungelösten Feststoffpartikel unmittelbar im Moment des ersten Einwirkens der aufzuwendenden Kräfte erfolgt.

26. Verfahren nach einem der Ansprüche 23 oder 24, **dadurch gekennzeichnet, dass** bei Verwendung von Hochdruckhomogenisatoren zur Erreichung einer mittleren PCS-Partikelgröße unterhalb von 1000 nm die Zahl der Homogenisationszyklen weniger als 10, insbesondere weniger als 5, bevorzugt weniger als 3 und speziell nur 1 beträgt.

27. Verfahren nach einem der Ansprüche 1 bis 26, **dadurch gekennzeichnet, dass** die Partikel, die in der in e) erhaltene Partikelsuspension enthalten sind, abgetrennt oder getrocknet, insbesondere lyophilisiert werden.

28. Verfahren nach einem der Ansprüche 1 bis 27, **dadurch gekennzeichnet, dass** die erhaltene Suspension oder die nach Abtrennung aus der Suspension erhaltenen Partikel zu Zwischen- oder Endprodukten weiterverarbeitet wird/werden.

29. Verfahren nach einem der Ansprüche 1 bis 28, **dadurch gekennzeichnet, dass** die erhaltene Suspension oder die nach Abtrennung aus der Suspension erhaltenen Partikel durch Aufbringen auf Zuckerpellets oder durch Einarbeitung in Matrixpellets weiterverarbeitet wird/werden.

30. Verfahren nach einem der Ansprüche 1 bis 26, **dadurch gekennzeichnet, dass** die erhaltene Suspension sprühgetrocknet oder lyophilisiert wird.

31. Verfahren nach einem der Ansprüche 1 bis 30, **dadurch gekennzeichnet, dass** die Feststoffzerkleinerung in e) mittels Hochdruckhomogenisation erfolgt und gleichzeitig eine Oberflächenmodifikation der entstehenden Feststoffpartikel bewirkt wird, wobei
e1) die Hochdruckhomogenisaton in Gegenwart eines fest vorliegenden Schutzkolloids 1 (Polyelektrolyt 1) erfolgt,
e2) nach Erreichen der gewünschten Partikelgröße der Feststoffpartikel durch Anwendung der erforderlichen Zahl von Homogenisationszyklen der erhaltenen Nanosuspension ein zweites, zu dem Schutzkolloid 1 (Polyelektrolyt 1) entgegengesetzt geladenes, fest vorliegendes Schutzkolloid 2 (Polyelektrolyt 2) zugesetzt wird und
e3) die resultierende Suspension erneut hochdruckhomogenisiert wird, bis eine fein verteilte, homogene, stabile Nanosuspension erhalten wird, wobei anschließend gegebenenfalls
e4) die in der erhaltenen homogenen, stabilen Nanosuspension enthaltenen Partikel durch Abtrennung gewonnen werden.

32. Verfahren nach Anspruch 31, **dadurch gekennzeichnet, dass** es sich bei den mittels Hochdruckhomogenisation hergestellten Feststoffnanopartikeln, die zum Zwecke der Oberflächenmodifikation und Stabilisierung mit mindestens zwei, bei einem bestimmten pH-Wert des Dispersionsmediums entgegengesetzt geladenen Polyelektrolytenschichten überzogenen sind, um Arzneistoffnanokristalle handelt.

33. Verfahren nach Anspruch 31 oder 32, **dadurch gekennzeichnet, dass** die Modifizierung der Oberfläche der Feststoffnanopartikel durch einen ersten Überzug bestehend aus mindestens einem bei einem bestimmten pH-Wert des Dispersionsmediums als Polykation vorliegenden ersten Polyelektrolyten und einen zweiten Überzug aus einem bei einem bestimmten pH-Wert des Dispersionsmediums als Polyanion vorliegenden zweiten Polyelektrolyten erreicht wird.

34. Verfahren nach einem der Ansprüche 31 bis 33, **dadurch gekennzeichnet, dass** es sich bei den eingesetzten Polyelektrolyten um Polymethacrylate, Celluloseacetatphthalat (CAP), Hydroxypropylmethylcellulosephthalat (HPMCP), Hydroxypropylmethylcelluloseacetatsuccinat (HPMCAS), Polyacrylsäure, Alginsäure, Carboxymethylcellulose, Dextransulfat, Ligninsulfonsäure, Polyvinylsulfonsäure, Polyvinylphosphonsäure, Chondroitinsulfonsäure, Gelatine A, Gelatine B, Chitosan, Protmainsulfat, Hyaluronsäure, Polylysinsäure, Polymilchsäure, Carragenane, Pektine, Gummi Arabicum, Nucleinsäuren, Polyethylenimin, Polyvinylamin und Polyvinylpyridin, sowie jeweils deren verschiedene Salze, freie Basen bzw. freie Säuren handelt.

35. Verfahren nach einem der Ansprüche 31 bis 34, **dadurch gekennzeichnet, dass** die resultierenden oberflächenmodifizierten Wirkstoffnanopartikel ein Zetapotential, gemessen in Wasser mit einer Leitfähigkeit im Bereich von 50µS, bei pH Werten zwischen 4 bis 7, im Bereich von 5 mV bis 100 mV, bevorzugt im Bereich von 20 mV bis 80 mV, besonders bevorzugt im Bereich von 30 mV bis 60 mV, besitzen, wobei ausschließlich der Betrag des Zetapotentials und nicht dessen Vorzeichen relevant ist.

36. Verwendung von gemäß einem der Ansprüche 1 bis 35 erhaltenen Partikelsuspensionen oder nach Abtrennung aus den Partikelsuspensionen erhaltenen Partikeln zur Herstellung von pharmazeutischen und kosmetischen Präparaten, vorzugsweise in Form von Tabletten und Kapseln, Cremes, Salben oder Pulvern zur Rekonstitution vor der Anwendung.

37. Verwendung nach Anspruch 36, **dadurch gekennzeichnet, dass** die erhaltene Suspension als Granulierungsflüssigkeit eingesetzt wird und das durch den Granulierungsschritt erhaltenen Granulat gegebenfalls vor der Anwendung zu Tabletten verpresst wird.

38. Verwendung nach Anspruch 36 oder 37, **dadurch gekennzeichnet, dass** die erhaltene Suspensionen oder Partikel in Hart- oder Weichgelatinekapseln gefüllt zur Anwendung kommen.

39. Verwendung nach einem der Ansprüche 36 bis 38, **dadurch gekennzeichnet, dass** die erhaltene Partikelsuspensionen oder Partikel in den Bereichen Nahrungsmittel, Textil, Agrar, insbesondere als Pestizidsuspensionen zur Anwendung kommen.

## Claims

1. Method for the production of ultrafine particle suspensions under mild conditions,
**characterised in that**
a) a solid substance insoluble in water or poorly soluble in water is dissolved in a suitable solvent,
b) the solution from a) is then frozen with the formation of a solid matrix,
c) optionally, the solvent is removed from the solid matrix in the frozen state formed in b) by drying, in particular lyophilisation,
d) the solid matrix formed in b), which has optionally been dried, in particular lyophilised, in accordance with c), is dispersed in the frozen state in a dispersion medium, and
e) medium to high forces are then applied to the dispersion produced in d) prior to the melting of the frozen, dispersed, solid matrix, so that a particle suspension is formed, the average particle size of which, determined by photon correlation spectroscopy (PCS), lies below 1000 nm, in particular in the range from 50 to <1000 nm, preferably below 800 nm, preferably in the range from 50 to 600 nm, and in particular below 400, preferably in the range from 50 to 200 nm, and especially below 100 nm.

2. Method as claimed in claim 1, **characterised in that** the solid to be dissolved is a drug active substance, a cosmetic active substance, an additive for foodstuffs, a dye or a pigment.

3. Method as claimed in any one of claims 1 or 2, **characterised in that** the medium to high forces are shear, cavitation, milling and/or ultrasonic forces, which are in particular applied by high-pressure homogenisers, jet-stream devices, rotor-stator colloid mills, ball mills, high-shear mixers or ultrasound devices, wherein the device used preferably operates at a power density of 10⁶ to 10¹³/m³, in particular in the range from 10⁹ to 10¹³/m³.

4. Method as claimed in any one of claims 1 to 3, **characterised in that** the solvents used for the dissolution of the solid substance which is insoluble in water or poorly soluble in water are hydrophilic liquids, in particular alcohols, preferably methanol, ethanol and isopropanol, mixtures of water with liquids completely or partially miscible with water, or hydrophilic liquids, in particular alcohols, preferably methanol, ethanol or isopropanol or other organic solvents, or liquids immiscible with water, in particular chloroform or dichloromethane, wherein preferred solvents are N-methyl-2-pyrrolidinone, 2-pyrrolidone, dimethyl-acetamide, ethanol, acetone, chloroform, dichloromethane, dimethyl sulphoxide, n-propanol, glycerol, ethylene glycol, dimethylformamide, dimethylacetamide or acids and bases, in particular hydrochloric acid, sulphuric acid, acetic acid, formic acid, triethanolamine, pyridine and ammonia, wherein optionally a mixture of two or more of the same is used.

5. Method as claimed in any one of claims 1 to 4, **characterised in that** the solid substance solution produced in a) also contains one or more further additives and dispersion-stabilising substances, in particular surfactants, stabilisers of the antiflocculant and polymer type, and inert fillers, wherein the concentrations per component, based on the weight, are preferably in the range from 1-90%, in particular from 1-20% and preferably below 10% and ideally below 0.01-5%.

6. Method as claimed in claim 5, **characterised in that** the stabilising substances include compounds from the series including the poloxamers, poloxamines, ethoxylated mono-and diglycerides, ethoxylated lipids and lipoids, ethoxylated fatty alcohols and alkylphenols, ethoxylated fatty acid esters, polyglycerine ethers and esters, lecithins, esters and ethers of sugars or sugar alcohols with fatty acids or fatty alcohols, phospholipids and sphingolipids, sterols, esters or ethers thereof and mixtures thereof of these compounds.

7. Method as claimed in claim 5, **characterised in that** the stabilising substances include egg lecithin, soya lecithin or hydrogenated lecithin, mixtures thereof or mixtures of one or both lecithins with one or more phospholipid components, cholesterol, cholesterol palmitate, stigmasterol or other sterols.

8. Method as claimed in claim 5, **characterised in that** the stabilisers include diacetyl phosphate, phosphatidyl-glycerol, saturated or unsaturated fatty acids, sodium cholate, peptising agents or amino acids.

9. Method as claimed in any one of claims 5 to 8, **characterised in that** the solid substance solution contains one or more viscosity-increasing substances, in particular cellulose ethers and esters, polyvinyl derivatives, alginates, xanthans, pectins, polyacrylates, poloxamers and poloxamines, polyvinyl alcohol or polyvinylpyrrolidone.

10. Method as claimed in any one of claims 5 to 9, **characterised in that** the solid substance solution in addition contains one or more further additives, in particular sugars or sugar alcohols, preferably glucose, mannose, trehalose, mannitol and sorbitol, fructose, sodium citrate, sodium hydrogen phosphate, sodium dihydrogen phosphate, sodium chloride, potassium chloride, glycerine, dyes or pigment.

11. Method as claimed in any one of claims 1 to 10, **characterised in that** for the freezing process in b) methods are used whereby complete freezing of the precise part of the produced solution to be frozen is effected within less than 60 seconds, preferably less than 30 seconds, particularly preferably less than 10 seconds, and especially less than 1 second.

12. Method as claimed in any one of claims 1 to 12 *[sic],* **characterised in that** the solvent is removed by lyophilisation from the solid frozen matrix, which is produced by temperature lowering in b), prior to dispersion in the dispersion medium forming the external phase, in particular with the use of solvents unsuitable for direct application to man and animals.

13. Method as claimed in any one of claims 1 to 12, **characterised in that** the drying is effected gently and slowly over several hours, preferably less than 168 hours, particularly preferably less than 72 hours, in particular less than 24 hours, in special cases less than 12 hours, in a suitable lyophilisation apparatus at reduced pressures, preferably at 0.5 mbar, particularly preferably at 0.1 mbar, in particular at 0.05 mbar, and at temperatures of preferably below 20°C, in particular below 0°C and especially below -20°C.

14. Method as claimed in any one of claims 1 to 13, **characterised in that** the solid matrix obtained after the removal of the solvent or solvents contains the solid substance in crystalline, partly crystalline or amorphous form and contains a residual content of solvent, based on the weight, of less than 50 percent, preferably less than 10 percent, particularly preferably less than 5 percent, and especially less than 1 percent.

15. Method as claimed in any one of claims 1 to 14, **characterised in that** the solid matrix obtained after the removal of the solvent is dispersed in a dispersion medium, in particular by stirring with paddle stirrers, rotor-stator systems or static mixers, so that a dispersion is obtained.

16. Method as claimed in claim 15, **characterised in that** water, mixtures of water and water-miscible liquids, non-aqueous media or organic solvents or lipophilic liquids, in particular oils and fatty oils, in which the solid substance is poorly soluble or insoluble, are used as the dispersion medium.

17. Method as claimed in any one of claims 1 to 16, **characterised in that** the dispersion produced in d) can also contain one or more further additives and one or more dispersion-stabilising substances, in particular surfactants, stabilisers of the antiflocculant and polymer type, and inert fillers, wherein the concentrations per component, based on the weight, preferably lie in the range from 1-90%, in particular from 1-20% and preferably below 10%, and ideally below 0.01-5%.

18. Method as claimed in claim 17, **characterised in that** the dispersion-stabilising substances include compounds from among the poloxamers, poloxamines, ethoxylated mono- and diglycerides, ethoxylated lipids and lipoids, ethoxylated fatty alcohols and alkylphenols, ethoxylated fatty acid esters, polyglycerine ethers and esters, lecithins, esters and ethers of sugars or sugar alcohols with fatty acids or fatty alcohols, phospholipids and sphingolipids, sterols, esters or ethers thereof and mixtures thereof of these compounds.

19. Method as claimed in claim 17, **characterised in that** the dispersion-stabilising substances include egg lecithin, soya lecithin or hydrogenated lecithin, mixtures thereof or mixtures of one or both lecithins with one or more phospholipid components, cholesterol, cholesterol palmitate, stigmasterol or other sterols.

20. Method as claimed in claim 17, **characterised in that** the stabilisers include dicetyl phosphate, phosphatidyl-glycerol, saturated or unsaturated fatty acids, sodium cholate, peptising agents or amino acids.

21. Method as claimed in any one of claims 1 to 20, **characterised in that** viscosity-increasing substances, in particular cellulose ethers and esters, polyvinyl derivatives, alginates, xanthans, pectins, polyacrylates, poloxamers and poloxamines, polyvinyl alcohol or polyvinyl-pyrrolidone, are contained in the dispersion.

22. Method as claimed in any one of claims 1 to 21, **characterised in that** the dispersion also contains additives such as sugars or sugar alcohols, in particular glucose, mannose, trehalose, mannitol and sorbitol, fructose or additives such as sodium citrate, sodium hydrogen phosphate, sodium dihydrogen phosphate, sodium chloride, potassium chloride, calcium chloride, glycerine.

23. Method as claimed in any one of claims 1 to 22, **characterised in that** the applied energy is applied by a high-pressure process, wherein in particular homogenisers of the piston-gap type (e.g. APV Gaulin, NiroSoavi, Avestin), of the jet-stream type (e.g. Microfluidizer) or a French Press (SLM Instruments, Urbana, US-A) are used.

24. Method as claimed in any one of claims 1 to 23, **characterised in that** when using high-pressure homogenisers the homogenisation pressure lies above 100 bar, preferably at or above 500 bar, in particular at or above 1500 bar and most favourably at or above 2000 bar.

25. Method as claimed in any one of claims 1 to 24, **characterised in that** when dispensing with the drying of the frozen matrix, this is dispersed in the frozen state in an external phase and the forces act on the dispersed still frozen matrix, so that melting of the frozen matrix and release of the undissolved solid substance particles, which is associated therewith, takes place directly at the moment of the first impact of the forces to be applied.

26. Method as claimed in any one of claims 23 or 24, **characterised in that** when using high-pressure homogenisers to achieve an average PCS particle size below 1000 nm the number of homogenisation cycles is less than 10, in particular less than 5, preferably less than 3 and especially only 1.

27. Method as claimed in any one of claims 1 to 26, **characterised in that** the particles which are contained in the particle suspension obtained in e) are separated or dried, in particular lyophilised.

28. Method as claimed in any one of claims 1 to 27, **characterised in that** the suspension obtained or the particles obtained after separation from the suspension is/are further processed to form intermediate or end products.

29. Method as claimed in any one of claims 1 to 28, **characterised in that** the suspension obtained or the particles obtained after separation from the suspension is/are further processed by application onto sugar pellets or by incorporation into matrix pellets.

30. Method as claimed in any one of claims 1 to 26, **characterised in that** the suspension obtained is spray-dried or lyophilised.

31. Method as claimed in any one of claims 1 to 30, **characterised in that** the solid substance comminution in e) is effected by high-pressure homogenisation and simultaneously a surface modification of the resulting solid substance particles is carried out, wherein
e1) the high-pressure homogenisation is effected in the presence of a protective colloid 1 (polyelectrolyte 1) in solid form,
e2) after achieving the desired particle size of the solid substance particles by application of the necessary number of homogenisation cycles, a second protective colloid 2 (polyelectrolyte 2) in solid form, charged oppositely to the protective colloid 1 (polyelectrolyte 1), is added to the nanosuspension obtained and
e3) the resulting suspension is again high-pressure homogenised until a finely divided, homogeneous, stable nanosuspension is obtained, wherein then optionally
e4) the particles contained in the homogenous, stable nanosuspension obtained are recovered by separation.

32. Method as claimed in claim 31, **characterised in that** the solid substance nanoparticles produced by high-pressure homogenisation, which for the purpose of surface modification and stabilisation are coated with at least two polyelectrolyte layers, oppositely charged when the dispersion medium is at a certain pH value, are drug substance nanocrystals.

33. Method as claimed in claim 31 or 32, **characterised in that** the modification of the surface of the solid substance nanoparticles is achieved by a first coating consisting of at least one first polyelectrolyte present as a polycation when the dispersion medium is at a certain pH value, and a second coating of a second polyelectrolyte present as a polyanion when the dispersion medium is at a certain pH value.

34. Method as claimed in any one of claims 31 to 33, **characterised in that** the polyelectrolytes used are polymethacrylates, cellulose acetate phthalate (CAP), hydroxypropylmethylcellulose phthalate (HPMCP), hydroxypropylmethylcellulose acetate succinate (HPMCAS), polyacrylic acid, alginic acid, carboxymethylcellulose, dextran sulphate, ligninsulphonic acid, polyvinylsulphonic acid, polyvinyl-phosphonic acid, chondroitinsulphonic acid, gelatine A, gelatine B, chitosan, Protmain sulphate, hyaluronic acid, polylysine acid, polylactic acid, carragenans, pectins, gum Arabic, nucleic acids, polyethylenimine, polyvinyl-amine and polyvinylpyridine, and respectively the various salts, free bases or free acids thereof.

35. Method as claimed in any one of claims 31 to 34, **characterised in that** the resulting surface-modified active substance nanoparticles have a zeta potential, measured in water with a conductivity in the range from 50µS, at pH values between 4 to 7, in the range from 5 mV to 100 mV, preferably in the range from 20 mV to 80 mV, particularly preferably in the range from 30 mV to 60 mV, wherein exclusively the value of the zeta potential and not its sign is relevant.

36. Use of particle suspensions obtained in accordance with any one of claims 1 to 35 or of particles obtained after separation from the particle suspensions for the production of pharmaceutical and cosmetic preparations, preferably in the form of tablets and capsules, creams, ointments or powders for reconstitution before use.

37. Use as claimed in claim 36, **characterised in that** the suspension obtained is used as a granulation liquid and the granulate obtained by the granulation step is optionally compressed into tablets before use.

38. Use as claimed in claim 36 or 37, **characterised in that** the suspensions or particles obtained are used being introduced into hard or soft gelatine capsules.

39. Use as claimed in any one of claims 36 to 38, **characterised in that** the particle suspensions or particles obtained are used in the foodstuffs, textile and agriculture fields, in particular as pesticide suspensions.

## Revendications

1. Procédé de production non agressif de suspensions de particules très fines, **caractérisé en ce que**
a) on dissout un solide non soluble dans l'eau ou difficilement soluble dans l'eau dans un solvant approprié,
b) la solution du point a) est ensuite congelée en formant une matrice solide,
c) éventuellement la matrice solide formée au point b) à l'état congelée est dépourvue du solvant par séchage, notamment, lyophilisation,
d) la matrice solide formée au point b) qui a été éventuellement séchée, notamment lyophilisée selon le point c) est dispersée dans un dispersant à l'état congelé, et
e) après la dispersion réalisée au point d), avant la fusion de la matrice solide dispersée congelée, des forces moyennes à élevées sont appliquées de sorte qu'une suspension de particule soit produite, dont la taille moyenne de particules déterminée par spectroscopie à corrélation de photons (PCS) est inférieure à 1000 nm, en particulier, est de l'ordre de 50 à < 1000 nm, de préférence inférieure à 800 nm, de préférence est de l'ordre de 50 à 600 nm et en particulier, inférieure à 400, de préférence de l'ordre de 50 à 200 nm et spécifiquement inférieure à 100 nm.

2. Procédé selon la revendication 1, **caractérisé en ce que** le solide à dissoudre est un principe actif médicamenteux, un principe actif cosmétique, un additif alimentaire, un colorant ou un pigment.

3. Procédé selon l'une des revendications 1 ou 2, **caractérisé en ce que** les forces moyennes à élevées sont des forces de cisaillement, de cavitation, de broyage et/ou d'ultrasons qui sont appliquées en particulier par des homogénéisateurs à haute pression, des appareils Jet-Stream, des broyeurs colloïdes rotor - stator, des broyeurs à billes, des mélangeurs à cisaillement élevé ou des appareils à ultrasons, l'appareil utilisé fonctionnant de préférence avec une densité de puissance de 10⁶ à 10¹³/m³, en particulier dans une plage de 10⁹ à 10¹³/m³.

4. Procédé selon l'une des revendications 1 à 3, **caractérisé en ce que** les solvants utilisés pour la dissolution des solides insolubles dans l'eau ou difficilement solubles dans l'eau sont des liquides hydrophiles, en particulier des alcools, de préférence le méthanol, l'éthanol et l'isopropanol, des mélanges d'eau avec des liquides complètement ou partiellement miscibles dans l'eau ou des liquides hydrophiles, en particulier des alcools, de préférence, le méthanol, l'éthanol ou l'isopropanol ou d'autres solvants organiques ou des liquides non miscibles dans l'eau, en particulier, le chloroforme ou le dichlorométhane, les solvants préférés étant la N-méthyl-2-pyrrolidinone, la 2-pyrrolidone, le diméthylacétamide, l'éthanol, l'acétone, le chloroforme, le dichlorométhane, le diméthylsulfoxyde, la N-propanol, le glycérol, l'éthylène glycol, le diméthylformamide, le diméthylacétamide ou des acides et des bases, en particulier l'acide chlorhydrique, l'acide sulfurique, l'acide acétique, l'acide formique, la triéthanolamine, la pyridine, l'ammoniaque, un mélange de deux ou plusieurs de ceux-ci étant éventuellement utilisé.

5. Procédé selon l'une des revendications 1 à 4, **caractérisé en ce que** la solution de solide produite au point a) contient encore un ou plusieurs autres adjuvants et substances stabilisant la dispersion, en particulier des tensioactifs, des stabilisants du type anti-floculants et polymères, et des substances de charge inertes, les concentrations par composant, par rapport au poids, se situant de préférence dans une plage de 1 à 90 %, en particulier de 1 à 20 % et de préférence, à moins de 10 %, et étant idéalement inférieures à 0,01-5 %.

6. Procédé selon la revendication 5, **caractérisé en ce que** les substances stabilisantes comprennent des composés de type poloxamères, poloxamines, mono- et diglycérides éthoxylés, lipides et lipoïdes éthoxylés, alcools gras et alkylphénols éthoxylés, esters d'acides gras éthoxylés, éthers et esters de polyglycérine, lécithines, esters et éthers de sucres ou de polyalcools avec des acides gras ou des alcools gras, phospholipides et sphingolipides, stérines, leurs esters ou éthers et les mélanges de ces composés.

7. Procédé selon la revendication 5, **caractérisé en ce que** les substances stabilisantes comprennent la lécithine d'oeuf, la lécithine de soja ou la lécithine hydrogénée, leurs mélanges ou les mélanges d'une ou deux lécithines avec un ou plusieurs composants phospholipide, cholestérol, palmitate de cholestérol, stigmastérine ou autres stérines.

8. Procédé selon la revendication 5, **caractérisé en ce que** les stabilisants comprennent le phosphate de diacétyle, le phosphatidyl-glycérol, des acides gras saturés ou insaturés, le cholate de sodium, des additifs peptisants ou des acides aminés.

9. Procédé selon l'une des revendications 5 à 8, **caractérisé en ce que** la solution de solide contient une ou plusieurs substances augmentant la viscosité, en particulier des éthers et esters de cellulose, des dérivés de polyvinyle, des alginates, des gommes xanthane, de la pectine, des polyacrylates, des poloxamères et des poloxamines, de l'alcool de polyvinyle, de la polyvinylpyrrolidone.

10. Procédé selon l'une des revendications 5 à 9, **caractérisé en ce que** la solution de solide contient en outre un ou plusieurs autres adjuvants, en particulier du sucre ou des polyalcools, de préférence le glucose, le mannose, le tréhalose, le mannitol et le sorbitol, le fructose, le citrate de sodium, l'hydrogénophosphate de sodium, le dihydrogénophosphate de sodium, le chlorure de sodium, le chlorure de potassium, la glycérine, des colorants ou pigments.

11. Procédé selon l'une des revendications 1 à 10, **caractérisé en ce que** pour le processus de congélation du point b), on utilise des procédés permettant d'effectuer une congélation complète de la partie qui doit justement être congelée de la solution produite en moins de 60 secondes, de préférence en moins de 30 secondes, de manière particulièrement préférée, en moins de 10 secondes, spécifiquement, en moins de 1 seconde.

12. Procédé selon l'une des revendications 1 à 12, **caractérisé en ce que** l'on retire le solvant de la matrice congelée à l'état solide obtenue par baisse de température au point b) avant la dispersion dans l'agent dispersant formant la phase externe par lyophilisation, en particulier par l'utilisation de solvants non appropriés pour l'utilisation directe chez l'être humain et l'animal.

13. Procédé selon l'une des revendications 1 à 12, **caractérisé en ce que** le séchage s'effectue de façon non agressive et lente sur plusieurs heures, de préférence en moins de 168 heures, de manière particulièrement préférée, en moins de 72 heures, en particulier en moins de 24 heures, dans des cas spécifiques, en moins de 12 heures, à une température de lyophilisation appropriée, à basse pression, de préférence à 0,5 mbar, de manière particulièrement préférée, à 0,1 mbar, en particulier à 0,05 mbar et à des températures de préférence inférieures à 20° C, en particulier de moins de 0° C et spécifiquement, inférieures à -20° C.

14. Procédé selon l'une des revendications 1 à 13, **caractérisé en ce que** la matrice solide obtenue par retrait du ou des solvants contient le solide sous forme cristalline, partiellement cristalline ou amorphe et une teneur résiduelle en solvant par rapport au poids, inférieure à 50 %, de préférence inférieure à 10 %, de manière particulièrement préférée, inférieure à 5 %, spécifiquement, inférieure à 1 %.

15. Procédé selon l'une des revendications 1 à 14, **caractérisé en ce que** la matrice solide obtenue après le retrait du solvant est dispersée dans un milieu de dispersion, en particulier par agitation avec des agitateurs à pâles, des systèmes rotor-stator ou des mélangeurs statiques de sorte que l'on obtienne une dispersion.

16. Procédé selon la revendication 15, **caractérisé en ce que** l'on utilise comme milieu de dispersion, de l'eau, des mélanges d'eau et de liquides miscibles dans l'eau, des milieux non aqueux ou des solvants organiques ou des liquides lipophiles, en particulier, des huiles et des huiles grasses, dans lesquels le solide est difficilement soluble ou est insoluble.

17. Procédé selon l'une des revendications 1 à 16, **caractérisé en ce que** la dispersion produite au point d) peut encore contenir un ou plusieurs autres adjuvants et une ou plusieurs autres substances stabilisant la dispersion, en particulier, des tensioactifs, des stabilisants du type des anti-floculants et des polymères tels que des agents de charge inertes, les concentrations par composant par rapport au poids étant de préférence de l'ordre de 1 à 90 %, en particulier, de 1 à 20 % et de préférence inférieures à 10 %, idéalement, inférieures à 0,01-5 %.

18. Procédé selon la revendication 17, **caractérisé en ce que** les substances stabilisant la dispersion comprennent des composés de la série des poloxamères, des poloxamines, des mono- et diglycérides éthoxylés, des lipides et lipoïdes éthoxylés, des alcools gras et des alkylphénols éthoxylés, des esters d'acides gras, éthers et esters de polyglycérine éthoxylés, des lécithines, des esters et éthers de sucres ou d'alcools de sucre avec des acides gras ou des alcools gras, des phospholipides et des sphingolipides, des stérines, leurs esters ou éthers et les mélanges de ces composés.

19. Procédé selon la revendication 17, **caractérisé en ce que** les substances stabilisant la dispersion comprennent la lécithine d'oeuf, la lécithine de soja ou la lécithine hydrogénée, leurs mélanges ou les mélanges d'une ou deux lécithines avec un ou plusieurs composants phospholipide, cholestérol, palmitate de cholestérol, stigmastérine ou autres stérines.

20. Procédé selon la revendication 17, **caractérisé en ce que** les stabilisants comprennent le phosphate de diacétyle, le phosphatidyl-glycérol, des acides gras saturés ou insaturés, le cholate de sodium, des additifs peptisants ou des acides aminés.

21. Procédé selon l'une des revendications 1 à 20, **caractérisé en ce que** la dispersion contient des substances augmentant la viscosité, en particulier des éthers et esters de cellulose, des dérivés de polyvinyle, des alginates, des gommes xanthane, de la pectine, des polyacrylates, des poloxamères et des poloxamines, de l'alcool de polyvinyle, de la polyvinylpyrrolidone.

22. Procédé selon l'une des revendications 1 à 21, **caractérisé en ce que** la dispersion contient en outre des adjuvants tels que du sucre ou des polyalcools, de préférence le glucose, le mannose, le tréhalose, le mannitol et le sorbitol, le fructose ou des adjuvants tels que le citrate de sodium, l'hydrogénophosphate de sodium, le dihydrogénophosphate de sodium, le chlorure de sodium, le chlorure de potassium, le chlorure de calcium, la glycérine.

23. Procédé selon l'une des revendications 1 à 22, **caractérisé en ce que** l'énergie déployée est utilisée par un processus à haute pression, dans lequel on utilise en particulier des homogénéisateurs du type à piston (par exemple, APV Gaulin, NiroSoavi, Avestin), du type Jet-Stream (par exemple, Microfluidifiant) ou French Press (SLM Instruments, Urbana, USA).

24. Procédé selon l'une des revendications 1 à 23, **caractérisé en ce que**, lors de l'utilisation d'homogénéisateurs à haute pression, la pression d'homogénéisation est supérieure à 100 bars, de préférence, supérieure ou égale à 500 bars, en particulier, supérieure ou égale à 1500 bars et le plus favorablement, supérieure ou égale à 2000 bars.

25. Procédé selon l'une des revendications 1 à 24, **caractérisé en ce que**, si l'on renonce au séchage de la matrice congelée, celle-ci est dispersée à l'état congelé dans une phase externe et les forces agissent sur la matrice encore congelée dispersée de telle sorte qu'il se produise une fusion de la matrice congelée et une libération correspondante des particules solides non dissoutes, immédiatement au moment de l'effet initial des forces à déployer.

26. Procédé selon l'une des revendications 23 ou 24, **caractérisé en ce que**, lors de l'utilisation d'homogénéisateurs à haute pression, pour obtenir une taille de particules moyenne inférieure à 1000 nm, le nombre de cycles d'homogénéisation est inférieur à 10, de préférence, inférieur à 5, de préférence, inférieur à 3 et spécifiquement, il est uniquement de 1.

27. Procédé selon l'une des revendications 1 à 26, **caractérisé en ce que** les particules qui sont contenues dans la suspension de particules obtenue au point e), sont séparées ou séchées, en particulier, lyophilisées.

28. Procédé selon l'une des revendications 1 à 27, **caractérisé en ce que** la suspension obtenue ou les particules obtenues après séparation de la suspension est/sont encore transformée(s) en produits intermédiaires ou produits finaux.

29. Procédé selon l'une des revendications 1 à 28, **caractérisé en ce que** la suspension obtenue ou les particules obtenues après séparation de la suspension est/sont encore transformée(s) par application sur des pastilles de sucre et/ou par incorporation dans des pastilles formant une matrice.

30. Procédé Selon l'une des revendications 1 à 26, **caractérisé en ce que** la suspension obtenue est séchée par pulvérisation ou lyophilisée.

31. Procédé selon l'une des revendications 1 à 30, **caractérisé en ce que** le broyage des solides du point e) s'effectue par homogénéisation à haute pression, ce qui entraîne en même temps une modification de surface des particules solides obtenues, sachant que
e1) l'homogénéisation à haute pression s'effectuant en présence d'un colloïde protecteur 1 se présente en phase solide (poly-électrolyte 1),
e2) après obtention des particules solides à la taille de particules souhaitée par utilisation du nombre nécessaire de cycles d'homogénéisation de la nanosuspension obtenue, on ajoute un deuxième colloïde protecteur 2 (poly-électrolyte 2) se présentant sous forme solide, chargé de façon opposée au colloïde protecteur 1 (poly-électrolyte 1) et
e3) la suspension obtenue est à nouveau homogénéisée à haute pression jusqu'à ce que l'on obtienne une nanosuspension stable, homogène, finement répartie, enfin, éventuellement
e4) les particules contenues dans la nanosuspension stable, homogène, pouvent être obtenues par séparation.

32. Procédé selon la revendication 31, **caractérisé en ce que** les nanoparticules solides produites par homogénéisation à haute pression qui sont revêtues aux fins de modification de surface et de stabilisation avec au moins deux couches de poly-électrolytes chargés de façon opposée à un pH déterminé du milieu de dispersion sont des nanocristaux de médicaments.

33. Procédé selon la revendication 31 ou 32, **caractérisé en ce que** la modification de la surface des nanoparticules solides est réalisée par un premier revêtement constitué d'au moins d'un premier poly-électrolyte se présentant sous forme de poly-cation à un pH déterminé du milieu de dispersion et d'un deuxième revêtement d'un deuxième poly-électrolyte se présentant sous forme d'un poly-anion à un pH déterminé du milieu de dispersion.

34. Procédé selon l'une des revendications 31 à 33, **caractérisé en ce que** les poly-électrolytes utilisés sont des polyméthacrylates, l'acétate phtalate de cellulose (CAP), le phtalate d'hydroxypropylméthylcellulose (HPMCP), l'acétate succinate d'hydroxypropylméthylcellulose (HPMCAS), l'acide polyacrylique, l'acide alginique, la carboxyméthylcellulose, le sulfate de dextrane, l'acide lignine-sulfonique, l'acide polyvinylsulfonique, l'acide polyvinylphosphonique, l'acide chondroïtine-sulfonique, la gélatine A, la gélatine B, le chitosan, le sulfate de protamine, l'acide hyaluronique, l'acide polylysique, l'acide polylactique, le carragheen, la pectine, la gomme arabique, les acides nucléiques, la polyéthylène-imine, la polyvinylamine et la polyvinylpyridine et respectivement, leurs sels, bases libres ou acides libres divers.

35. Procédé selon l'une des revendications 31 à 34, **caractérisé en ce que** les nanoparticules de principe actif obtenues, modifiées en surface, ont un potentiel zêta mesuré dans l'eau ayant une capacité conductrice de l'ordre de 50 µs à des pH de 4 à 7, de l'ordre de 5 mV à 100 mV, de préférence de l'ordre de 20 mV à 80 mV, de manière particulièrement préférée de l'ordre de 30 mV à 60 mV, le niveau du potentiel zêta exclusivement et non ses signes étant pertinents.

36. Utilisation de suspensions de particules obtenues selon l'une des revendications 1 à 35 ou des particules obtenues après séparation de suspensions de particules pour la production de préparations pharmaceutiques et cosmétiques, de préférence sous la forme de comprimés et de capsules, de crèmes, de pommades ou de poudres à reconstituer avant utilisation.

37. Utilisation selon la revendication 36, **caractérisée en ce que** la suspension obtenue est utilisée sous forme de liquide de granulation et le granulat obtenu par l'étape de granulation est éventuellement comprimé avant l'utilisation en comprimés.

38. Utilisation selon la revendication 36 ou 37, **caractérisée en ce que** les suspensions ou particules obtenues sont conditionnées dans des capsules de gélatine dure ou souple pour utilisation.

39. Utilisation selon l'une des revendications 36 à 38, **caractérisée en ce que** les suspensions de particules ou lés particules obtenues sont utilisées dans les domaines alimentaires, textiles, agricoles, en particulier comme suspensions pesticides.
